# EUROPEAN PATENT APPLICATION

(11) **EP 3 984 542 A1**
(43) Date of publication of application: **20.04.2022**
(21) Application number: 21199893.5
(22) Date of filing: 20.11.2015
(51) Int. Cl.: A61K 35/15, A61K 38/17, A61K 35/17, C07K 14/705, C07K 16/28, G01N 33/50, C12Q 1/6897

(54) **SYSTEMS AND METHODS FOR ASSESSING MODULATORS OF IMMUNE CHECKPOINTS**

(30) Priority: 20.11.2014 US 201462082458 P
(62) Divisional of application: 15860628.5
(71) Applicant: Promega Corporation, Madison, WI 53711-5399 (US)
(72) Inventor: Cong, Mei, Madison, 53711-5399 (US); Cheng, Zhijie Jey, Madison, 53711-5399 (US); Karassina, Natasha, Madison, 53711-5399 (US); Grailer, Jamison, Madison, 53711-5399 (US); Fan, Frank, Madison, 53711-5399 (US)
(74) Representative: Potter Clarkson

(57) **Abstract**

Provided herein are compositions, systems, and methods for assessing modulators of immune checkpoints. In particular, artificial antigen presenting cells (aAPCs) and immune effector cells are provided to assess the potency of test agents to inhibit immune checkpoints.

## Description

### CROSS REFERENCE TO RELATED APPLICATION

The present invention claims the priority benefit of U.S. Provisional Patent Application 62/082,458, filed November 20, 2014, which is incorporated by referenced in its entirety.

### FIELD

Provided herein are compositions, systems, and methods for assessing modulators of immune checkpoints. In particular, artificial antigen presenting cells (aAPCs) and immune effector cells are provided to assess the potency of test agents to inhibit immune checkpoints.

### BACKGROUND

Among the most promising approaches to activating therapeutic antitumor immunity is the blockade of immune checkpoints. Immune checkpoints refer to a plethora of inhibitory pathways hardwired into the immune system that are crucial for maintaining self-tolerance and modulating the duration and amplitude of physiological immune responses. It is now clear that tumors co-opt certain immune-checkpoint pathways as a major mechanism of immune resistance, particularly against T cells that are specific for tumor antigens. Because many of the immune checkpoints are initiated by ligand-receptor interactions, they can be blocked by agents that inhibit these interactions, for example antibodies specific for the ligand or receptor.

A ligand-receptor interaction that has been investigated as a target for cancer treatment is the interaction between the transmembrane programmed cell death 1 protein (PD-1; also known as CD279) and its ligand, PD-1 ligand 1 (PD-L1) and PD-1 ligand 2 (PD-L2). In normal physiology, PD-L1 and PD-L2 on the surface of a cell binds to PD-1 on the surface of an immune cell, which inhibits the activity of the immune cell. Upregulation of PD-L1 on the surface of some cancer cells may allow them to evade the host immune system by inhibiting T cells that might otherwise attack tumors. Antibodies that bind to either PD-1 or PD-L1, and therefore block the interaction, defeat the inhibitory mechanism and allow the immune cells to attack the cancer cells or tumor. Initial clinical trial results with an IgG4 PD-1 antibody called NIVOLUMAB have been published (Pardoll, DM (Mar 22, 2012). "The blockade of immune checkpoints in cancer immunotherapy." Nature reviews cancer. 12 (4): 252-64.; herein incorporated by reference in its entirety).

Traditional methods of measuring antibodies against immunoblockade receptors have numerous drawbacks. For example, animal models can be used to assess the antitumor effects of mAb blockage by phenotypic analysis of tumor masses in tumor-bearing mice, mean survival time, overall survival rate of mice, etc. Such analysis has the major drawbacks of being cost and time prohibitive. As an alternative, freshly isolated peripheral blood mononuclear cells (PBMC) can be used to study immunotherapy drugs on inhibitory blockade of cytokine production. For example, cryopreserved PBMCs have been used in clinical studies, which reported with significantly reduced expression of both PD-1 and PD-L1 on PBMC-derived CD3+/CD8+ T cells and CD45+/CD14+ monocytes obtained from adult control subjects. However, the isolation of PBMCs is tedious and such experiments produce great variability in results. Finally, cell-based assays have been developed to measure IL-2 production (ELISA based assay) in Jurkat cells over expressing immune checkpoint receptors, however such assays require a minimum of two days after antibody stimulation to perform. Convenient methods for measuring the effectiveness of antibodies against immune checkpoint receptors are needed.

### SUMMARY

Provided herein are compositions, systems, and methods for assessing modulators of immune checkpoints. In particular, artificial antigen presenting cells (aAPCs) and immune effector cells are provided to assess the potency of test agents (e.g., antibodies) to inhibit immune checkpoints.

In some embodiments, provided herein are compositions comprising an artificial antigen presenting cell (aAPC) or phospholipid droplet, displaying on its surface: (1) an antigen responsive element activator, and (2) an immune checkpoint ligand (ICL). In some embodiments, the antigen responsive element activator is a T cell receptor (TCR) complex activator. In some embodiments, compositions are provided comprising an artificial antigen presenting cell (aAPC) or phospholipid droplet displaying on its surface a T cell receptor (TCR) activator and an immune checkpoint ligand (ICL). Although embodiments described herein are typically described in reference to the TCR complex and a TCR activator, the compositions, systems, and methods described herein may also be applied to other antigen responsive elements and activators thereof. In some embodiments, the aAPC further displays on its surface a second (or third, fourth, etc.), different immune checkpoint ligand.

In some embodiments, TCR activator is a surface displayed molecular entity (e.g., protein, peptide, polypeptide, etc.) that binds to, or otherwise interacts with a component of the TCR complex to activate the TCR complex and TCR-dependent pathways. In some embodiments, the TCR activator is an anti-cluster of differentiation 3 antibody (anti-CD3), a membrane fusion protein containing an antibody fragment of anti-CD3, or antibody fragment thereof, major histocompatibility complex (MHC), etc.

In some embodiments, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some embodiments, the ICL is any suitable immune checkpoint ligand including but not limited to, PD-L1, PD-L2, B7-H4, CD155, galectin-9, HVEM, etc.

In some embodiments, provided herein are compositions comprising an artificial antigen presenting cell (aAPC) displaying on its surface: (1) A TCR activator (e.g., anti-CD3) and (2) an immune checkpoint ligand (e.g., PD-L1).

In some embodiments, the artificial, exogenous, and/or engineered elements of an APC include, but are not limited to: a TCR activator (e.g., anti-CD3 antibody, MHC, etc.), an immune checkpoint ligand (e.g., PD-L1), etc.

In some embodiments, provided herein are compositions comprising artificial antigen presenting cells (aAPCs) or phospholipid droplets displaying on their surface a T cell receptor (TCR) activator and an immune checkpoint ligand (ICL). In some embodiments, upon interaction of the aAPC or phospholipid droplet with an effector cell comprising a TCR and an immune checkpoint receptor (ICR), the TCR activator activates the TCR of the effector cell, and the ICL interacts with the ICR forming an ICR/ICL complex. In some embodiments, formation of the ICR/ICL complex modulates the downstream effects of TCR activation. In some embodiments, the TCR activator is an anticluster-of-differentiation-3 (CD3) antibody, or antibody fragment thereof, or major histocompatibility complex (MHC). In some embodiments, the ICL is selected from the group consisting of PD-L1, PD-L2, B7-H4, CD155, galectin-9, and HVEM.

In some embodiments, provided herein are systems comprising: (a) an artificial antigen presenting cell or phospholipid droplet displaying on its surface: (i) a T cell receptor (TCR) activator and (ii) an immune checkpoint ligand (ICL); and (b) an artificial effector cell displaying on its surface an immune checkpoint receptor (ICR) and comprising: (i) a TCR complex and (ii) a reporter (e.g., a TCR-pathway-dependent reporter) of one or more of: (A) ICR/ICL complex formation, (B) TCR activation, and/or (C) the TCR activation pathway-dependent reporter. In some embodiments, formation of an ICR/ICL complex results in a detectable alteration in signal (e.g., change in activity, change in expression, etc.) from the TCR-activation-pathway-dependent reporter (e.g., versus in the absence of ICR/ICL interaction). In some embodiments, systems further comprise a blockade agent or test blockade agent. In some embodiments, the blockade agent or test blockade agent inhibits formation of the ICR/ICL complex resulting in inhibition of the signal from the TCR activation pathway-dependent reporter. In some embodiments, the alteration of the signal from the TCR activation pathway-dependent reporter is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).

In some embodiments, the TCR activator is a surface displayed molecular entity (e.g., protein, peptide, polypeptide, etc.) that binds to, or otherwise interacts with, a component of the TCR complex to active the TCR complex and TCR-dependent pathways. In some embodiments, the TCR activator is anti-cluster of differentiation 3 antibody (anti-CD3) or antibody fragment thereof, major histocompatibility complex (MHC), etc.

In some embodiments, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some embodiments, the ICL is any suitable immune checkpoint ligand including, but not limited to, PD-L1, PD-L2, B7-H4, CD155, galectin-9, HVEM, etc.

In some embodiments, the artificial effector cell is a T cell selected from the group including, but not limited to, Jurkat cells, HuT-78, CEM, Molt-4, etc., that has been engineered to contain, express, etc., one or more elements (e.g., a reporter construct, exogenous gene, etc.) not native to the parent (e.g., non-artificial) cell.

In some embodiments, the ICR is any suitable immune inhibitory receptor including, but not limited to, PD-1, CTLA-4, LAG-3, TIM-3, TIGIT, CD160, BTLA, IL-10 receptor, etc.

In some embodiments, the TCR complex is an octomeric complex of variable TCR receptor α and β chains with three dimeric signaling modules CD3δ/ε, CD3γ/ε, and CD247 ζ/ζ or ζ/η.

In some embodiments, the reporter (e.g., TCR activation pathway-dependent reporter) is an element on or within an effector cell having a characteristic (e.g., activity, expression, concentration, localization, interactions, modification, etc.) which is dependent upon, or correlates with, one or more of (i) ICR/ICL complex formation, (ii) TCR activation, and/or (iii) activation of the TCR signaling pathway. In some embodiments, the reporter is downstream of ICR/ICL complex formation and/or TCR activation (e.g., an engineered reporter construct, a detectable element/signal/interaction naturally occurring within said effector cell). In some embodiments, a reporter is an element on or within the effector cell having a characteristic (e.g., activity, expression, concentration, localization, interactions, modification, etc.) which is altered by one or more of (i) ICR/ICL complex formation, (ii) TCR activation, and/or (iii) the TCR signaling pathway. In some embodiments, the reporter is intrinsic to the effector cell (e.g., an intrinsic element of T cells, Jurkat cells, etc.). In such embodiments, alterations in characteristics of that intrinsic reporter are detectable as a signal of the presence, absence, and or level of one or more of i) ICR/ICL complex formation, (ii) TCR activation, and/or (iii) the TCR signaling pathway activation. Exemplary intrinsic reporters and/or signals include, but are not limited to: gene expression (e.g., of an TCR-pathway-dependent gene), protein concentration (e.g., a protein expressed by a TCR-pathway-dependent gene), a protein-protein interaction (e.g., mediated by ICR/ICL complex formation and/or TCR activation), activity of a TCR-dependent protein (e.g., an enzyme, proteinase, kinase), localization and movement of an intrinsic effector cell element, etc. In some embodiments, the reporter is an extrinsic to the effector cell (e.g., a reporter element or construct that has been introduced or engineered into the effector cell). In such embodiments, alterations in a characteristic of the extrinsic and/or engineered reporter are detectable as a signal of the presence, absence, and or level of one or more of: (i) ICR/ICL complex formation, (ii) TCR activation, and/or (iii) the TCR signaling pathway activation. Exemplary extrinsic reporters and/or signals include, but are not limited to: expression of a TCR-dependent and/or TCR-pathway-dependent reporter construct (e.g., under the control of an NFAT-response element, NF-κB-response element, AP1-response element, a promoter containing one or multiple of the mentioned response elements, IL-2 promoter), activity of a reporter whose activation and/or expression is TCR- or TCR-pathway dependent (e.g., luciferase, beta lactamase, CAT, SEAP, fluorescent protein, etc.), a protein-protein interaction, protein movement, endogenous gene up-regulation detection via qPCR, etc.

In some embodiments, provided herein are systems comprising: (a) an artificial antigen presenting cell or phospholipid droplet displaying on its surface: (i) a T cell receptor (TCR) activator, (ii) a first immune checkpoint ligand (ICL), and (iii) a second ICL; and (b) an artificial effector cell displaying on its surface: (i) a first immune checkpoint receptor (ICR), (ii) a second ICR, and (iii) a TCR complex, and comprising a reporter (e.g., a TCR-pathway-dependent reporter) of one or more of: (A) complex formation between the first ICR and the first ICL, (B) complex formation between the second ICR and the second ICL, (C) TCR activation, and/or (D) the TCR activation pathway-dependent reporter. In some embodiments, formation of a complex between the first ICR and first ICL and/or second ICR and second ICL results in a detectable alteration in signal (e.g., change in activity, change in expression, etc.) from the TCR-activation-pathway-dependent reporter (e.g., versus in the absence of an ICR/ICL interaction). In some embodiments, systems further comprise a blockade agent or test blockade agent. In some embodiments, the blockade agent or test blockade agent inhibits formation of the complex between the first ICR and first ICL and/or second ICR and second ICL resulting in inhibition of the signal from the TCR activation pathway-dependent reporter. In some embodiments, the alteration of the signal from the TCR activation pathway-dependent reporter is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).

In some embodiments, provided herein are systems comprising: (a) an effector cell displaying T cell Receptor (TCR) and an immune checkpoint receptor (ICR) on its surface, and a TCR activation pathway-dependent reporter (e.g., nuclear factor of activated T cells (NFAT) promoter-dependent reporter); and (b) an artificial antigen presenting cell (aAPC) displaying a TCR activator (e.g., surface expressed anti-cluster of differentiation 3 antibody or antibody fragments, major histocompatibility complex (MHC), etc.) and an immune checkpoint ligand (ICL); wherein the ICR and ICL form an ICR/ICL complex upon interaction. In some embodiments, formation of the ICR/ICL complex results in a suppressed expression of the NFAT-promoter-dependent reporter. In some embodiments, systems further comprise a blockade agent or test blockade agent. In some embodiments, the blockade agent or test blockade agent inhibits formation of the ICR/ICL complex, resulting in an increased expression of the NFAT-promoter-dependent reporter. In some embodiments, increased expression is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein). In some embodiments, increased expression is a 6-12 fold increase over suppressed expression. In some embodiments, the effector cell is a T cell including, but not limited to, Jurkat cells, HuT-78, CEM, Molt-4, etc. In some embodiments, the reporter is a luciferase, a beta lactamase, CAT, SEAP, a fluorescent protein, a gene product (e.g., detected by qPCR, mass spectrometry, etc.), etc. In some embodiments, the ICR is any immune inhibitory receptor including but not limited to PD-1, CTLA-4, LAG-3, TIM-3, TIGIT, CD160, BTLA, IL-10 receptor, etc., and the ICL is any corresponding immune inhibitory ligand including, but not limited to, PD-L1, PD-L2, B7-H4, CD155, galectin-9, HVEM, etc. In some embodiments, the blockade agent or test blockade agent is an antibody (or antibody fragment), protein, peptide, or small molecule, vaccine, or combination thereof.

In some embodiments, provided herein are systems comprising: (a) an effector cell comprising TCR and PD-1 on its surface and a TCR activation pathway-dependent (e.g., NFAT-promoter-dependent) reporter; and (b) an artificial antigen presenting cell displaying a TCR activator (e.g., surface expression of anti-cluster of differentiation 3 antibody or antibody fragments, major histocompatibility complex (MHC), etc.) and PD-L1 on its surface. In some embodiments, formation of a PD-1/PD-L1 complex results in a suppressed expression of the TCR activation pathway-dependent (e.g., NFAT-promoter-dependent) reporter. In some embodiments, systems further comprise a blockade agent or test blockade agent. In some embodiments, the blockade agent or test blockade agent inhibits formation of the PD-1/PD-L1 complex, resulting in an increased expression of the TCR activation pathway-dependent reporter. In some embodiments, increased expression is at least 2-fold (e.g., 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 50-fold, 100-fold, 200-fold, 500-fold, 1000-fold, 2000-fold, 5000-fold, and any ranges therein).
In some embodiments, the reporter is a luciferase, a beta lactamase, CAT, SEAP, a fluorescent protein, a protein-protein interaction, etc. In some embodiments, expression is detected by directly measuring the amount of gene expression (e.g., by qPCR, by mass spectrometry, etc.).

In some embodiments, provided herein are compositions comprising an artificial effector cell displaying a TCR and any immune inhibitory receptor including, but not limited to, PD-1, CTLA-4, LAG-3, TIM-3, TIGIT, CD160, BTLA, IL-10 receptor, etc., on its surface and comprising a TCR activation pathway-dependent reporter (e.g., NFAT-promoter-dependent reporter) such as a luciferase, a beta lactamase, CAT, SEAP, a fluorescent protein, etc.

In some embodiments, provided herein are systems comprising: (a) an effector cell displaying on its surface an immune checkpoint receptor (ICR) and comprising a T cell receptor (TCR); and (b) an artificial antigen presenting cell (aAPC) displaying on its surface a T cell receptor (TCR) activator and an immune checkpoint ligand (ICL); wherein the ICR and ICL form an ICR/ICL complex upon interaction. In some embodiments, formation of the ICR/ICL complex results in modulation of TCR activation by the TCR activator and/or modulation of one or more TCR-dependent pathways. In some embodiments, modulation comprises: (a) inhibition of TCR activation by the TCR activator and/or one or more TCR-dependent pathways; or (b) enhancement of TCR activation by the TCR activator and/or one or more TCR-dependent pathways. In some embodiments, the TCR activator is an anti-cluster-of-differentiation-3 (CD3) antibody, or antibody fragment thereof, or major histocompatibility complex (MHC). In some embodiments, the ICL is selected from the group consisting of PD-L1, PD-L2, B7-H4, CD155, galectin-9, and HVEM. In some embodiments, the ICR is selected from the group consisting of PD-1, CTLA-4, LAG-3, TIM-3, CD160, TIGIT, IL-10 receptor, and BTLA. In some embodiments, the effector cell is selected from the group consisting of a Jurkat, HuT-78, CEM, and Molt-4. In some embodiments, the effector cell further comprises a reporter of: TCR activation, TCR pathway activation, and/or ICR/ICL complex modulation of TCR activation or TCR pathway activation. In some embodiments, the reporter characteristic comprises: cellular concentration, expression, activity, localization, or protein-protein interactions. In some embodiments, formation of the ICR/ICL complex modulates said characteristic. In some embodiments, the reporter is a natural reporter, e.g., intrinsic to the effector cell type, having a characteristic which is detectable and correlates to TCR activation, TCR pathway activation, and/or ICR/ICL complex modulation of TCR activation or TCR pathway activation. In some embodiments, the reporter is an artificial reporter, e.g., exogenous to the effector cell type, having a characteristic which is detectable and correlates to TCR activation, TCR pathway activation, and/or ICR/ICL complex modulation of TCR activation or TCR pathway activation. In some embodiments, the reporter is a gene, the expression of which is under the control of TCR-pathway-dependent reporter. In some embodiments, the TCR-pathway-dependent reporter is a nuclear factor of activated T cells (NFAT) promoter. In some embodiments, the reporter comprises a gene coding for a protein selected from the group consisting of a luciferase, a beta lactamase, CAT, SEAP, a fluorescent protein, or a quantifiable gene product. In some embodiments, the system further comprises a blockade agent or test blockade agent. In some embodiments, the blockade agent inhibits formation of the ICR/ICL complex, resulting in modulation of TCR activation or TCR pathway activation. In some embodiments, the blockade agent or test blockade agent is a small molecule, peptide, protein, or antibody.

In some embodiments, provided herein are systems comprising: (a) an effector cell comprising TCR and PD-1 on its surface and an NFAT-promoter-dependent luciferase; and (b) an artificial antigen presenting cell (aAPC) displaying on its surface: anti-CD3 antibody or antibody fragment thereof and PD-L1. In some embodiments, formation of a PD-1/PD-L1 complex results in a suppressed expression of the NFAT-promoter-dependent luciferase. In some embodiments, systems further comprise a blockade agent or test blockade agent. In some embodiments, the blockade agent or test blockade agent inhibits formation of the PD-1/PD-L1 complex resulting in an increased expression of the NFAT-promoter-dependent luciferase.

In some embodiments, provided herein are methods comprising: (a) contacting: (i) an artificial antigen presenting cell (aAPC) displaying on its surface: (A) a T cell receptor (TCR) activator and (B) an immune checkpoint ligand (ICL) with (ii) an effector cell comprising a TCR complex and displaying on its surface an immune checkpoint receptor (ICR), wherein interaction of the TCR activator with the TCR complex activates a TCR signaling pathway, wherein said ICR and said ICL form an ICR/ICL complex upon interaction, and wherein formation of the ICR/ICL complex enhances or inhibits TCR activation and/or the TCR signaling pathway; and (b) detecting the presence, absence, and/or level of TCR activation in said effector cell. In some embodiments, the methods further comprise: (c) contacting said effector cell and/or said aAPC with a blockade agent or test blockade agent. In some embodiments, the methods further comprise: (d) detecting the effect of said blockade agent or test blockade agent on (i) ICR/ICL complex formation, (ii) TCR activation, and/or (iii) the TCR signaling pathway. In some embodiments, one or more of (i) ICR/ICL complex formation, (ii) TCR activation, and/or (iii) the TCR signaling pathway are detected in the presence and absence of said blockade agent or test blockade agent to determine an effect.

In some embodiments, (i) ICR/ICL complex formation, (ii) TCR activation, and/or (iii) the TCR signaling pathway are detected based upon a reporter downstream of ICR/ICL complex formation and/or TCR activation (e.g., an engineered reporter construct, a detectable element/signal/interaction naturally occurring within said effector cell). In some embodiments, a reporter is an element on or within the effector cell having a characteristic (e.g., activity, expression, concentration, localization, interactions, modification, etc.) of which is altered by one or more of (i) ICR/ICL complex formation, (ii) TCR activation, and/or (iii) the TCR signaling pathway. In some embodiments, the reporter is intrinsic to the effector cell (e.g., an intrinsic element of T cells, Jurkat cells, etc.). In such embodiments, alterations in a characteristic of that intrinsic reporter are detectable as a signal of the presence, absence, and or level of one or more of i) ICR/ICL complex formation, (ii) TCR activation, and/or (iii) the TCR signaling pathway activation. Exemplary intrinsic reporters and/or signals include, but are not limited to: gene expression (e.g., of a TCR-pathway-dependent gene), protein concentration (e.g., a protein expressed by a TCR-pathway-dependent gene), a protein-protein interaction (e.g., mediated by ICR/ICL complex formation and/or TCR activation), TCR-pathway-dependent protein modification, activity of a TCR-dependent protein, localization of an intrinsic effector cell element, etc. In some embodiments, the reporter is extrinsic to the effector cell (e.g., a reporter element or construct that has been introduced or engineered into the effector cell). In such embodiments, alterations in characteristics of the extrinsic and/or engineered reporter are detectable as a signal of the presence, absence, and or level of one or more of: (i) ICR/ICL complex formation, (ii) TCR activation, and/or (iii) the TCR signaling pathway activation. Exemplary extrinsic reporters and/or signals include, but are not limited to: expression of a TCR-dependent and/or TCR-pathway-dependent reporter construct (e.g., under the control of an NFAT promoter), activity of a reporter whose activation and/or expression is TCR- or TCR-pathway dependent (e.g., luciferase, beta lactamase, CAT, SEAP, fluorescent protein, etc.), a protein-protein interaction, protein movement, detection of protein modification, endogenous gene up-regulation detection via qPCR, etc.

In some embodiments, the TCR activator is a surface displayed molecular entity (e.g., protein, peptide, polypeptide, etc.) that binds to, or otherwise interacts with, a component of the TCR complex to activate the TCR complex and TCR-dependent pathways. In some embodiments, the TCR activator is anti-cluster of differentiation 3 antibody (anti-CD3) or antibody fragments thereof, major histocompatibility complex (MHC), etc.

In some embodiments, the ICL is a molecular entity (e.g., protein, peptide, polypeptide, etc.) that interacts with an immune checkpoint receptor (ICR) on an effector cell (e.g., T cell, Jurkat, etc.) to modulate (e.g., inhibit, enhance, etc.) the immune response of the effector cell. In some embodiments, the ICL is any suitable immune checkpoint ligand including but not limited to PD-L1, PD-L2, B7-H4, CD155, galectin-9, HVEM, etc.

In some embodiments, the effector cell is a T cell selected from the group including, but not limited to, Jurkat cells, HuT-78, CEM, Molt-4, etc. In some embodiments, the effector cell is artificial (e.g., engineered to contain, express, etc. one or more elements (e.g., a reporter construct, exogenous gene, etc.) not native to the parent (e.g., non-artificial) cell). In some embodiments, the effector cell is an unengineered human or animal T cell.

In some embodiments, the ICR is any suitable immune inhibitory receptor including, but not limited to, PD-1, CTLA-4, LAG-3, TIM-3, TIGIT, CD160, BTLA, IL-10 receptor, etc.

In some embodiments, the TCR complex is an octomeric complex of variable TCR receptor α and β chains with three dimeric signaling modules CD3δ/ε, CD3γ/ε, and CD247 ζ/ζ or ζ/η.

In some embodiments, provided herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface and a TCR activation pathway-dependent reporter; (ii) an artificial antigen presenting cell (aAPC) displaying an anti-cluster of differentiation 3 (CD3) antibody, or antibody fragment thereof, and an immune checkpoint ligand, wherein the ICR and ICL form an ICR/ICL complex upon interaction; and (iii) a test blockade agent; (b) incubating the mixture in (a) to allow signal induction; (c) detection of said signal from said reporter; and (d) comparing said signal from the cell co-culture system with and without the test blockade agent, wherein a gain of signal indicates inhibition of said ICR/ICL complex by said test blockade agent. In some embodiments, the effector cell is a T cell including, but not limited to, Jurkat cells, HuT-78, CEM, Molt-4, etc. In some embodiments, the reporter is a luciferase, a beta lactamase, CAT, SEAP, a fluorescent protein, etc. In some embodiments, the ICR can be any immune inhibitory receptor including, but not limited to, PD-1, CTLA-4, LAG-3, TIM-3, TIGIT, CD160, BTLA, IL-10 receptor, etc., and the ICL can be any immune inhibitory ligand including, but not limited to, PD-L1, PD-L2, B7-H4, CD155, galectin-9, HVEM, etc. In some embodiments, the blockade agent or test blockade agent is an antibody (or antibody fragment), protein, peptide, or small molecule or combination thereof.

In some embodiments provided herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface, and a TCR activation pathway-dependent reporter (e.g., NFAT-promoter dependent reporter), and (ii) an artificial antigen presenting cell (aAPC) displaying a TCR activator (e.g., an anti-cluster of differentiation 3 (CD3) antibody or antibody fragment thereof) and an immune checkpoint ligand (ICL), wherein the ICR and ICL form an ICR/ICL complex upon interaction; (b) detecting a signal from said reporter; (c) adding said test blockade agent; (d) repeating detection of said signal from said reporter; and (e) comparing said signal from step (b) with said signal from step (d), wherein a gain of signal from step (b) to step (d) indicates inhibition of said ICR/ICL complex by said test blockade agent. In some embodiments, the effector cell is a T cell including, but not limited to, Jurkat cells, HuT-78, CEM, Molt-4, etc. In some embodiments, the reporter is a luciferase, a beta lactamase, CAT, SEAP, a fluorescent protein, gene expression (e.g., quantified by qPCR), etc. In some embodiments, the ICR can be any immune inhibitory receptor including, but not limited to, PD-1, CTLA-4, LAG-3, TIM-3, TIGIT, CD160, BTLA, IL-10 receptor, etc., and the ICL can be any corresponding immune inhibitory ligand including, but not limited to, PD-L1, PD-L2, B7-H4, CD155, galectin-9, HVEM, etc. In some embodiments, the blockade agent or test blockade agent is an antibody (or antibody fragment), protein, peptide or small molecule or combination thereof. In some embodiments, the ICR is PD-1, and the ICL is PD-L1. In some embodiments, the test blockade agent is an antibody or a small molecule.

In some embodiments, provided herein are methods of measuring the potency of a test blocking agent to inhibit the interaction of a immune checkpoint receptor and an immune checkpoint ligand, comprising: (a) co-culturing: (i) an effector cell displaying T Cell Receptor (TCR) and an immune checkpoint receptor (ICR) on its surface, and a TCR activation pathway-dependent reporter (e.g., NFAT-promoter dependent reporter), and (ii) an artificial antigen presenting cell (aAPC) displaying a TCR activator (e.g., an anti-cluster of differentiation 3 (CD3) antibody or antibody fragment thereof) and an immune checkpoint ligand (ICL), wherein the ICR and ICL form an ICR/ICL complex upon interaction; (b) detecting a signal from said reporter in the presence of a test blockade agent; and (c) comparing said signal from step (b) with a control (e.g., signal in the absence of said test blockade agent, a control signal, background, a zero control, a negative control, etc.), wherein a gain of signal relative to control indicates inhibition of said ICR/ICL complex by said test blockade agent. In some embodiments, methods further provide a step of adding the test blockage agent.

In some embodiments, provided herein are methods comprising: (a) forming a system comprising: (i) an effector cell displaying on its surface an immune checkpoint receptor (ICR), and comprising a T Cell Receptor (TCR) and a TCR-pathway-dependent reporter, and (ii) an artificial antigen presenting cell (aAPC) displaying on its surface a TCR activator and an immune checkpoint ligand (ICL); wherein the ICR and ICL form an ICR/ICL complex upon interaction, and wherein formation of the ICR/ICL complex results in modulation of TCR activation by the TCR activator and/or modulation of one or more TCR-dependent pathways; and (b) detecting said TCR-pathway-dependent reporter or a signal from said reporter. In some embodiments, methods further comprise adding to the system a blockade agent, wherein the blockade agent inhibits formation of the ICR/ICL complex or inhibits ICR/ICL-dependent modulation of TCR activation by the TCR activator and/or modulation of one or more TCR-dependent pathways. In some embodiments, the TCR-pathway-dependent reporter or a signal from said reporter is detected: (1) before, (2) concurrent with, and/or (3) after addition of said blockade agent. In some embodiments, systems further comprise a step of comparing signal from (1) before, (2) concurrent with, and/or (3) after addition of said blockade agent to determine the effect of the blockade agent.

In some embodiments, provided herein are methods comprising: (a) co-culturing:
(i) an effector cell displaying T Cell Receptor (TCR) and an immune checkpoint receptor (ICR) on its surface, and TCR-pathway-dependent reporter, and (ii) an artificial antigen presenting cell (aAPC) displaying a TCR activator and an immune checkpoint ligand (ICL), wherein the ICR and ICL form an ICR/ICL complex upon interaction; (b) adding said test blockade agent; (c) detecting said signal from said reporter; and (d) comparing said signal from said reporter with a control, wherein a gain of signal with respect to the control indicates inhibition of said ICR/ICL complex by said test blockade agent.
   In some embodiments, provided herein are methods of identifying an ICR/ICL blockade agent, comprising: (a) co-culturing: (i) an effector cell displaying T Cell Receptor (TCR) and an immune checkpoint receptor (ICR) on its surface, and TCR-pathway-dependent reporter, and
(ii) an artificial antigen presenting cell (aAPC) displaying anti-cluster of differentiation 3 (CD3) antibody, or antibody fragment thereof, and an immune checkpoint ligand, wherein the ICR and ICL form an ICR/ICL complex upon interaction; (b) detecting a signal from said reporter; (c) adding said test blockade agent; (d) repeating detection of said signal from said reporter; and (e) comparing said signal from step (b) with said signal from step (d), wherein a gain of signal from step (b) to step (d) indicates inhibition of said ICR/ICL complex by said test blockade agent.

In some embodiments, provided herein are methods of identifying an ICR/ICL blockade agent, comprising: (a) contacting: (i) an effector cell displaying on its surface PD-1, and comprising: T cell Receptor (TCR) and a nuclear factor of activated T cells (NFAT) promoter-dependent luciferase reporter, and (ii) an artificial antigen presenting cell (aAPC) displaying anti-cluster-of-differentiation-3 (CD3) antibody, or antibody fragment thereof, and PD-L1; and (b) detecting a signal from said NFAT-promoter-dependent luciferase reporter. In some embodiments, methods further comprise: (c) adding said test blockade agent; and (d) repeating detection of said signal from said reporter. In some embodiments, methods further comprise: (e) comparing said signal from step (b) with said signal from step (d), wherein a gain of signal from step (b) to step (d) indicates inhibition of said ICR/ICL complex by said test blockade agent.

In some embodiments, provided herein are methods comprising administering an artificial antigen presenting cell (aAPC) displaying on its surface a T cell receptor (TCR) activator and an immune checkpoint ligand (ICL) to a system comprising a natural effector cell.
In some embodiments, provided herein are methods comprising administering an artificial antigen presenting cell (aAPC) displaying on its surface a T cell receptor (TCR) activator and an immune checkpoint ligand (ICL) to a system comprising an artificial effector cell.

### BRIEF DESCRIPTION OF THE DRAWINGS

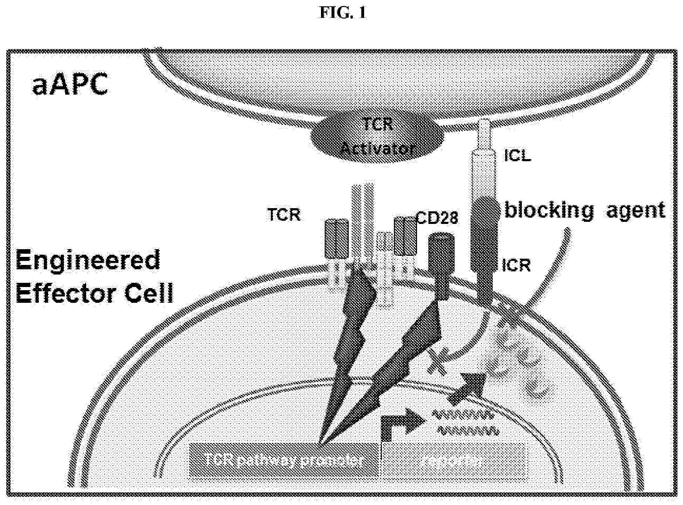

Figure 1 shows a schematic of a general system for assessing inhibitors of immune checkpoints. An aAPC displaying a T cell receptor (TCR) activator and immune checkpoint ligand (ICL) on its cell membrane is shown interacting with an engineered effector cell displaying an immune checkpoint receptor (ICR), CD28 and the TCR complex and comprising a TCR-pathway-promoter-dependent reporter construct. Upon co-culture of the aAPC and engineered effector cell, the TCR activator activates the TCR complex; however, the interaction of ICR and ICL inhibits activation of expression from the TCR pathway and little expression of the reporter occurs. In the presence of a blocking agent that inhibits the ICR/ICL interaction, the TCR pathway is activated, expression of the reporter form the TCR-pathway-promoter is enhanced, and reporter signal is elevated.

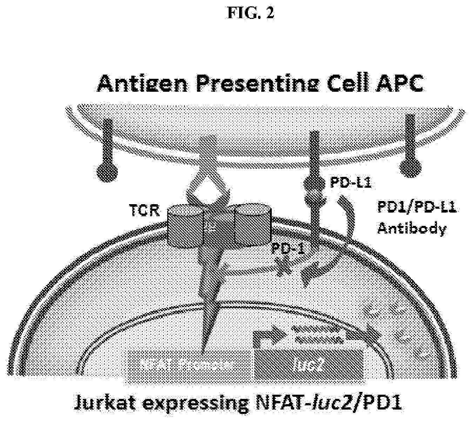

Figure 2 shows a schematic of an exemplary system for assessing inhibitors of immune checkpoints. An aAPC displaying PD-L1 and anti-CD3 antibody Fab domain on its cell membrane is shown interacting with a Jurkat T cell displaying PD-1, the TCR complex, and comprising NFAT-promoter-dependent luciferase reporter construct. Upon co-expression of the aAPC and Jurkat effector cell, anti-CD3 activates the TCR complex; however, the interaction of PD-1 and PD-L1 inhibits activation of expression from the NFAT promoter, and little expression of the luciferase occurs. In the presence of an inhibitor of the PD-1/PD-L1 interaction, the activated TCR complex activates expression of the luciferase reporter from the NFAT promoter, and luciferase signal is elevated.
Figure 3 shows a graph depicting the specificity of an exemplary PD-1/PD-L1 blockade assay.
Figure 4 shows graphs depicting concentration-dependent activation of reporter expression by an anti-PD-L1 antibody in an exemplary PD-1/PD-L1 blockade assay.
Figure 5 shows graphs depicting concentration-dependent activation of reporter expression by an anti-PD-1 antibody in an exemplary PD-1/PD-L1 blockade assay.
Figure 6 shows a schematic of system analogous to the one depicted in Figure 2, but the APC does not express TCR activator.
Figure 7 shows graphs depicting concentration-dependent activation of reporter expression by an anti-PD-L1 antibody in a PD-1/PD-L1 blockade assay with aAPCs lacking engineered membrane-bound anti-CD3.
Figure 8 show graphs depicting the absence of PD-1/PD-L1-dependent inhibition of T- cell activation by anti-CD3/PD-L1-Fc beads.
Figures 9A-9B show flow cytometry results confirming the attachment of PD-L1 to beads.
Figure 10 shows a graph demonstrating that anti-CD3:PD-L1 beads (1:9 ratio) failed to induce inhibition of T cell activation, and therefore that anti-PD-1/PD-L1 antibody failed to induce T cell activation.
Figure 11 shows a graph demonstrating that extended length anti-PD-L1-Fc on anti-CD3:PD-L1 beads failed to induce inhibition of T cell activation, and therefore that anti-PD-1/PD-L1 antibody failed to induce T cell activation.
Figure 12 shows a contemplated mechanistic explanation for the failure of anti-CD3:PD-L1 beads to perform like anti-CD3:PD-L1 aAPCs in exemplary blockade assays.
Figure 13 shows a schematic of an exemplary system for assessing inhibitors of immune checkpoint receptor PD-1 and its ligand, PD-L2.
Figures14A-14B show graphs depicting concentration-dependent activation of reporter expression by an anti-PD-1 antibody (b) or an anti-PD-L2 (a) antibody in an exemplary PD-1/PD-L2 blockade assay.
Figure 15 shows a schematic of an exemplary system for assessing inhibitors of immune checkpoint receptor CTLA-4 and its ligand, CD80/CD86.
Figure 16 shows a graph depicting concentration-dependent activation of reporter expression by an anti-CTLA-4 antibody in an exemplary CTLA-4 blockade assay.
Figure 17 shows a schematic of an exemplary system for assessing inhibitors of immune checkpoint receptor TIGIT and its ligand, CD155.
Figure 18 shows a graph depicting concentration-dependent activation of reporter expression by an anti-TIGIT antibody in an exemplary TIGIT/CD155 blockade assay using a TIGIT Effector Cells in Jurkat-T cells.
Figure 19 shows a schematic of an exemplary system for assessing inhibitors of immune checkpoint receptor TIGIT and its ligand, CD155.
Figure 20 shows a graph depicting concentration-dependent activation of reporter expression by an anti-TIGIT antibody in an exemplary TIGIT/CD155 blockade assay using a TIGIT Effector Cells in CEM T cells.
Figure 21 shows a schematic of an exemplary system for assessing inhibitors of immune checkpoint receptor PD-1 and its ligand PD-L1.
Figure 22 shows a graph depicting concentration-dependent IL-2 production by an anti-PD-1 antibody in an exemplary PD-1/PD-L1 blockade assay.
Figure 23 shows a schematic of an exemplary system for assessing inhibitors of immune checkpoint receptor LAG-3 and its ligand, MHC II.
Figure 24 shows graphs depicting concentration-dependent activation of reporter expression by an anti-LAG-3 antibody in an exemplary LAG-3 blockade assay.

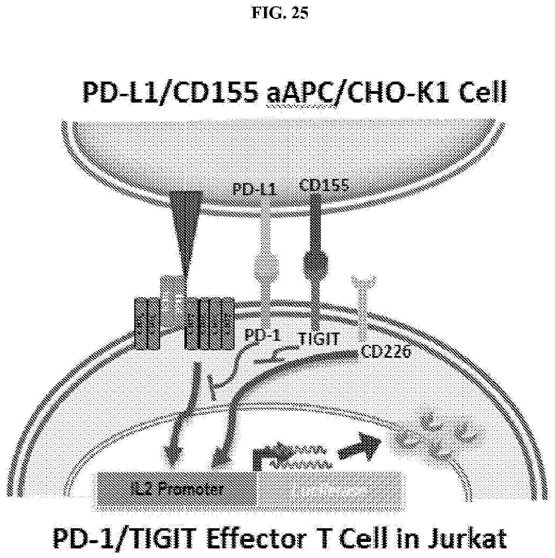

Figure 25 shows a schematic of an exemplary system for assessing inhibitors of immune checkpoint receptors TIGIT and PD-1 and their ligands, CD155 and PD-L1, respectively. An aAPC displaying PD-L1 and CD155 and anti-CD3 antibody Fab domain on its cell membrane is shown interacting with a Jurkat T cell displaying PD-1, co-stimulatory receptor CD226, TIGIT, the TCR complex, and comprising IL2-promoter-dependent luciferase reporter construct. Upon the interaction of the aAPC and Jurkat effector cell, anti-CD3 Ab activates the TCR complex and CD155 co-stimulates CD226. These lead to activation of IL-2 promoter driving luciferase expression; however, the interaction of PD-1 with PD-L1 leads to reduced TCR pathway activation and reduced IL-2 promoter-dependent reporter expression. In addition, CD155 has higher binding affinity with TIGIT than with CD226. Therefore, TIGIT inhibits interaction of CD155 with CD226 and leads to reduced reporter expression. In the presence of an inhibitor of the PD-1/PD-L1 and/or TIGIT/CD155 interaction, the expression of the luciferase reporter from IL-2 promoter is enhanced, and the luciferase signal is elevated.
Figure 26a-b shows graphs depicting concentration-dependent activation of reporter expression by (a) an anti-PD-1 antibody and (b) an anti-TIGIT antibody in an exemplary PD-1/TIGIT blockade assay using a PD-1/TIGIT Effector Cells in Jurkat-T cells and PD-L1/CD155 aAPC/CHO-K1 cells. Figure 26c shows the effects of a combination of anti-PD-1 and anti-TIGIT antibodies compared with either antibody alone.

### DEFINITIONS

As used herein, the term "immune checkpoint receptor" ("ICR") refers to a surface receptor protein on an immune cell (e.g., T cell, Jurkat cells, etc.) that modulates the immune activity of the cell when bound to its ligand. Of particular interest herein are "inhibitory immune checkpoint receptors" which inhibit cellular immune activity upon ligand binding to the receptor. Examples of "inhibitory immune checkpoint receptors" include, but are not limited to, PD-1, CTLA-4, LAG-3, TIM-3, CD160, TIGIT, IL-10 receptor, and BTLA.

As used herein, the term "immune checkpoint ligand" ("ICL") refers to a ligand of an immune checkpoint receptor. "Immune checkpoint ligands" are commonly surface-displayed proteins on antigen presenting cells (APCs). Through an interaction with an immune-cell-displayed immune checkpoint receptor, an "immune checkpoint ligand" modulates the immune response of the immune cell (e.g., T cell) to the antigen presenting cell. Examples of "immune checkpoint ligands" that bind inhibitory immune checkpoint receptors include, but are not limited to, PD-L1, PD-L2, B7-H4, CD155, galectin-9, HVEM, etc.

As used herein, the terms "immune checkpoint," "checkpoint pathway," and "immune checkpoint pathway" refer to a pathway by which the binding of an immune checkpoint ligand to an immune checkpoint receptor modulates the amplitude and quality of the activation of immune cells (e.g., T cells, Jurkat cells, HuT-78, CEM, Molt-4, etc.).

As used herein, the term "immune checkpoint blockade" refers to the inhibition of an immune checkpoint pathway by the administration or expression of a "blockade agent." Typically, the "blockade agent" prevents the interaction of the immune checkpoint receptor and ligand, thereby inhibiting the checkpoint pathway. A blockade agent may be a small molecule, peptide, antibody or fragment thereof, etc. that binds to an immune checkpoint ligand or immune checkpoint receptor and inhibits the formation of the ICR/ICL complex. A blockade agent may also function by preventing signaling by the ICR/ICL complex.

As used herein, the term "artificial," as in "artificial antigen presenting cell" or "artificial effecter cell," refers to an entity (e.g., cell, construct, etc.) that does not occur in nature. For example, a cell engineered to express an exogenous gene (e.g., reporter construct) that is not intrinsic to the parent (e.g., unengineered cell) is "artificial."

As used herein, the term "antibody" refers to a protein having one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad of immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. The basic immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

The term "antibody" is used herein in the broadest sense and specifically covers human, non-human (e.g. murine), and humanized monoclonal antibodies (including full length monoclonal antibodies), polyclonal antibodies, multi-specific antibodies (e.g., bispecific antibodies), single-chain antibodies, and antibody fragments so long as they exhibit the desired biological activity. Antibodies may exist as intact immunoglobulins, or as modifications in a variety of forms including, for example, FabFc₂, Fab, Fv, Fd, (FabN)₂, an Fv fragment containing only the light and heavy chain variable regions, a Fab or (Fab)N₂ fragment containing the variable regions and parts of the constant regions, a single-chain antibody, e.g., scFv, CDR-grafted antibodies and the like. The heavy and light chain of an Fv may be derived from the same antibody or different antibodies thereby producing a chimeric Fv region. The antibody may be of animal (especially mouse or rat) or human origin or may be chimeric or humanized. As used herein the term "antibody" includes these various forms.

The term "reporter" is used herein in the broadest sense to describe a molecular entity, a characteristic and/or property of which (e.g., concentration, amount, expression, activity, localization, interactions (e.g., protein-protein interactions), etc.) can be detected and correlated with a characteristic and/or property of a system containing the reporter (e.g., cell, cell lysate, in vitro system, organism, in vivo system, etc.). A "reporter" may be an intrinsic (e.g., endogenous) element of the system that exhibits one or more detectable and correlatable properties, or an artificial (e.g., exogenous) element engineered or introduced into the system, that exhibits a detectable characteristic linked to process (e.g., gene expression) or component within the system. Suitable reporters include, but are not limited to: intrinsic genes or proteins (e.g., expression, concentration, activity, or protein-protein interactions of which may be correlated to system (e.g., cellular processes)), exogenous genes or proteins (e.g., expression, concentration, activity, or protein-protein interactions of which may be correlated to system (e.g., cellular processes)), luciferases, beta lactamases, CAT, SEAP, fluorescent proteins, etc.

### DETAILED DESCRIPTION

Provided herein are compositions, systems, and methods for assessing modulators of immune checkpoints. In particular, artificial antigen presenting cells (aAPCs) and immune effector cells are provided to assess the potency of test agents (e.g., antibodies) to inhibit immune checkpoints.

In some embodiments, provided herein are compositions comprising an artificial antigen presenting cell (aAPC) comprising (e.g., displaying on its surface): (1) A TCR activator and (2) an immune checkpoint ligand (ICL).

An antigen-presenting cell (APC), or accessory cell, is a cell that processes and presents foreign antigens complexed with major histocompatibility complexes (MHCs) on its surface, in a process is known as antigen presentation. T cells may recognize these complexes using their T cell receptors (TCRs) and/or T cell receptor complexes. Artificial antigen presenting cells (aAPCs) are typically derived from primary or transformed human or xenogeneic cells that are engineered (e.g., using retroviral or lentiviral transduction) to introduce molecules that provide the co-stimulatory and adhesion properties required for immune synapse formation. This strategy allows stringent control of the delivery of the many positive and negative signals to T cells during the interaction with the aAPC (See, e.g., Turtle et al. (Cancer J. 2010 Jul-Aug; 16(4):374-81) for review of aAPCs in immunotherapy; herein incorporated by reference in its entirety).

In some embodiments, aAPCs are provided that express and/or display on their surface an activator of TCR. In some embodiments, interaction of a TCR activator with TCR results in activation of TCR-dependent pathways and enhanced gene expression from promoters downstream of TCR (e.g., NFAT-dependent expression). In some embodiments, aAPCs are provided that express anti-CD3 antibody (e.g., full antibody, Fab domain, etc.). In some embodiments, aAPCs are provided that display anti-CD3 antibody (e.g., full antibody, Fab domain, etc.) on their surface. It has been demonstrated that resting T-lymphocytes are activated by anti-CD3 antibodies (Tsoukas et al, J Immunol. 1985 Sep; 135(3): 1719-23.; herein incorporated by reference in its entirety). In some embodiments, anti-CD3-expressing aAPCs activate T cells (e.g., Jurkat cells) through the binding of anti-CD3 to surface-displayed CD3 within the TCR complex of the T cells (e.g., Jurkat cells). In some embodiments, anti-CD3 binding to the TCR complex results in a series of downstream biochemical events mediated by associated enzymes, co-receptors, specialized adaptor molecules, and activated or released transcription factors. For example, anti-CD3 binding to the TCR results in activation of NFAT proteins (e.g., NFATc1, NFATc2, NFATc3, NFATc4, NFAT5) and increased gene expression from NFAT promoters (e.g., promoters that bind NFAT resulting in enhanced transcription). In some embodiments, expression of an NFAT-promoter-linked reporter (e.g., luciferase) provides a quantitative measure of T cell activation and immune checkpoint blockade. In some embodiments, other downstream events triggered by the binding of anti-CD3 to the TCR provide suitable markers of T cell activation (or can be manipulated to provide markers of as much). Other examples of T cell activators (e.g., TCR activators) include superantigens, anti-TCR antibodies, anti-CD2 antibodies, anti-CD4 antibodies, PHA, MHC and cognate peptides, and Con A, any of which may be expressed in aAPCs and/or surface displayed thereon to activate effector cells.

In some embodiments, aAPCs are provided that express an ICL (e.g., for use in systems comprising effector cells displaying ICR). In some embodiments, aAPCs are provided that display ICL on their surface including, but not limited to, PD-L1, PD-L2, B7-H4, CD155, galectin-9, HVEM, etc. In some embodiments, aAPCs express and/or display a peptide/polypeptide with at least 50% sequence identity (e.g., >50%, >60%, >70%, >80%, >90%, >95%, or more) with all or a portion of ICL, and retain at least 50% (e.g., >50%, >60%, >70%, >80%, >90%, >95%, or more) of the affinity of ICL for ICR. In some embodiments, aAPCs are provided that display or express a peptide/polypeptide that has at least 50% (e.g., >50%, >60%, >70%, >80%, >90%, >95%, or more) of the affinity of ICL for ICR.

In some embodiments, aAPCs are provided that express PD-L1 (e.g., for use in systems comprising effector cells displaying PD-1). In some embodiments, aAPCs are provided that display PD-L1 on their surface. In some embodiments, aAPCs express and/or display a peptide/polypeptide with at least 50% sequence identity (e.g., >50%, >60%, >70%, >80%, >90%, >95%, or more) with all or a portion of ICL and retain at least 50% (e.g., >50%, >60%, >70%, >80%, >90%, >95%, or more) of the affinity of PD-L1. In some embodiments, aAPCs are provided that display or express a peptide/polypeptide that has at least 50% (e.g., >50%, >60%, >70%, >80%, >90%, >95%, or more) of the affinity of PD-L1 for PD-1.

In some embodiments, provided herein are systems comprising: (a) an artificial antigen presenting cell comprising (e.g., displaying on its surface): (i) a T cell receptor (TCR) activator, and (ii) an immune checkpoint ligand (ICL) (e.g., any of the aAPCs described in above); and (b) an artificial effector cell comprising an immune checkpoint receptor (ICR) (e.g., displayed on its surface) and comprising: (i) a TCR complex and (ii) a reporter (e.g., reporter of one or more of: (A) ICR/ICL complex formation, (B) TCR activation, and/or (C) the TCR signaling pathway TCR activation pathway-dependent reporter).

In certain embodiments, systems are provided comprising an aAPC (e.g., as described in Section I above or elsewhere herein) and an effector cell (e.g., artificial effector cell). An effector cell is a lymphocyte (e.g., T cell, Jurkat cell, etc.) that has been induced to differentiate into a form capable of mounting a specific immune response. In some embodiments, effector cells are provided that express and/or display surface proteins and/or complexes of surface proteins useful in the blockade assays described herein (e.g., an antigen-responsive element (e.g., TCR), ICR (e.g., PD-1), etc.). In some embodiments, an effector cell useful in the embodiments described herein has been engineered to express a reporter of effector cell activation. In some embodiments, one or more pathways involved in the activation of T cells activate the reporter and/or expression of the reporter, thereby detectably signaling blockade of the immune checkpoint and T cell activation.

In some embodiments, an effector cell displays numerous markers indicative of a T cell (e.g., human T cell, Jurkat cell, etc.). For example, an effector cell may display one or more of: the T cell receptor complex and/or components thereof (e.g., CD3, TCRα, TCRβ, TCRγ, TCRδ, etc.), CD4, CD8, CD28, CTLA-4, CD40L, CD2, LFA-1, etc.

Activation of T cells occurs through the simultaneous engagement of the T cell receptor and a co-stimulatory molecule on the T cell by the major histocompatibility complex (MHCII) peptide and co-stimulatory molecules on an antigen presenting cell (APC). Once properly engaged by the APC, downstream signaling pathways are initiated to activate the T cell. For example, co-stimulatory molecules engage the phosphoinositide 3-kinase (PI3K) pathway, generating phosphatidylinositol (3,4,5)-trisphosphate (PIP3) at the plasma membrane and recruiting PH-domain-containing signaling molecules (e.g., pyruvate dehydrogenase lipoamide kinase isozyme 1 (PDK1)) that are essential for the activation of PKCθ, and eventual IL-2 production. A non-limiting set of downstream effects of the activation include: phosphorylation of CD28, LAT and SLP-76, which allows the aggregation of signaling complexes around these proteins; recruitment of SLP-76 to the membrane, where it can then bring in PLC-γ, VAV1, Itk and potentially PI3K; activation of transcription factors, such as NF-κB and AP-1; activation of calcium receptors on the endoplasmic reticulum (ER); release of calcium from the ER into the cytosol which causes STIM1 clustering on the ER membrane and leads to activation of cell membrane CRAC channels that allow additional calcium to flow into the cytosol from the extracellular space; binding of calmodulin by aggregated cytosolic calcium, which then activates calcineurin; calcineurin activation of nuclear factor of activated T cells (NFAT), translocation of NFAT to the nucleus; NFAT activation of the transcription of a pleiotropic set of genes, most notably, IL-2, interferons, tumor necrosis factor, IL-17, IL-10, IL-4, IL-13, IL-23, IL-1, transforming growth factor beta, IL-8 and other chemokines, and numerous other pathways. In some embodiments, any of these downstream effects may find use as a marker of blockade of an immune checkpoint (e.g., by liking to downstream effect to a detectable reporter).

In some embodiments, systems and methods are provided for assessing the potency of blockade agents (e.g., antibodies) in inhibiting immune checkpoint pathways. Exemplary systems comprise: (1) an artificial effector cell (e.g., T cell (e.g., Jurkat cell, etc.), etc.) displaying an antigen-responsive element (e.g., T Cell Receptor (TCR) complex) and an immune checkpoint receptor (ICR) on its surface (e.g., PD-1) and comprising a reporter; (2) an artificial antigen presenting cell (aAPC) displaying on its surface an activator of the antigen responsive element of the effector cell (e.g., anti-CD3 antibody or a fragment thereof) and an immune checkpoint ligand (ICL) corresponding to the ICR of the effector cell (e.g., PD-L1); and (3) a test blockade agent (e.g., antibody to the ICL or ICR). In some embodiments, in the absence of the blockade agent (or in the event that the test blockade agent is ineffective), the ICR and ICL interact and modulate (e.g., inhibit or enhance) the signal from the reporter construct. However, in the presence of a potent blockade agent, the interaction of the ICR and ICL is inhibited and modulation the reporter signal by the ICR/ICL is inhibited.

In some embodiments, systems and methods are provided for assessing the potency of blockade agents (e.g., antibodies) in blocking inhibitory immune checkpoint pathways. Exemplary systems comprise: (1) an artificial effector cell (e.g., engineered T cell (e.g., Jurkat cell, etc.), etc.) displaying an antigen-responsive element (e.g., T Cell Receptor (TCR) complex) and an immune checkpoint receptor (ICR) on its surface and comprising a reporter construct, the expression of which is dependent upon activation of the antigen-responsive element; (2) an artificial antigen presenting cell (aAPC) displaying on its surface an activator of the antigen responsive element of the effector cell and an immune checkpoint ligand (ICL) corresponding to the ICR of the effector cell; and (3) a test blockade agent (e.g., antibody to the ICL or ICR). In some embodiments, activation of the antigen responsive element by interaction with the activator of the antigen responsive element results in a signal (or absence of signal) from the reporter. In some embodiments, in the absence of the blockade agent (or in the event that the test blockade agent is ineffective), the ICR and ICL interact and modulate the signal from the reporter. In the presence of a potent blockade agent, the interaction of the ICR and ICL is inhibited, modulation of the reporter signal by the ICR/ICL is blocked, and the reporter signal tends toward the signal in the absence of an ICR/ICL. In some embodiments, when effector cells and aAPCs are co-cultured, a first signal level is produced from the reporter (e.g., because the ICR/ICL complex modulates reporter signal); however, addition of a potent blockade agent results in a second signal level from the reporter (e.g., because formation of the ICR/ICL is inhibited, and therefore modulation of reporter signal by the ICR/ICL complex is reduced).

In some embodiments, systems and methods are provided for assessing the potency of blockade agents (e.g. antibodies) in inhibiting immune checkpoint pathways. Exemplary systems comprise: (1) an effector cell (e.g., T cell (e.g., Jurkat cell, etc.), etc.) displaying T Cell Receptor (TCR) complex and an immune checkpoint receptor (ICR) on its surface and expressing a NFAT-promoter-dependent reporter (e.g., luciferase); (2) an artificial antigen presenting cell (aAPC) displaying anti-CD3 antibody (or a fragment thereof) and an immune checkpoint ligand (ICL) corresponding to the ICR of the effector cell; and (3) a test blockade agent (e.g., antibody to the ICL or ICR). In some embodiments, in the absence of the blockade agent (or in the event that the test blockade agent is ineffective), the ICR and ICL interact and inhibit activation of the NFAT promoter by the CD3-antibody-bound TCR complex, thereby inhibiting expression of the reporter. However, in the presence of a potent blockade agent, the interaction of the ICR and ICL is inhibited, the NFAT promoter is activated, and the reporter is expressed. In some embodiments, when effector cells and aAPCs are co-cultured, little or no signal is produced from the reporter (e.g., because the ICR/ICL complex inhibits reporter expression); however, addition of a potent blockade agent results in increased expression from the NFAT promoter and a gain of signal from the reporter.

In some embodiments, systems and methods are provided for assessing the potency of blockade agents (e.g. antibodies) in inhibiting the PD-1/PD-L1 immune checkpoint pathway. Exemplary systems comprise: (1) a Jurkat cell displaying T Cell Receptor (TCR) complex and PD-1 on its surface and expressing an NFAT-promoter-induced luciferase reporter (e.g., luc2); (2) an artificial antigen presenting cell (aAPC) displaying anti-CD3 antibody and PD-L1 on its surface; and (3) a test blockade agent (e.g., antibody to PD-1 or PD-L1). In some embodiments, in the absence of the blockade agent (or in the event that the test blockade agent is ineffective), the PD-1 and PD-L1 interact and inhibit activation of the NFAT promoter by the anti-CD3-antibody-bound TCR complex; therefore, luciferase expression is inhibited, and the reporter signal is diminished. In the presence of a potent blockade agent, the interaction of the PD-1, and PD-L1 is inhibited, the NFAT promoter is activated, luciferase expression is enhanced, and the reporter signal increases. In some embodiments, when Jurkat effector cells and aAPCs are co-cultured, little or no luciferase is expressed, and little or no signal is generated (e.g., because the PD-1/PD-L1 complex inhibits reporter expression); however, addition of a potent blockade agent results in increased expression of luciferase and a gain of signal from the system.

In some embodiments, systems, and methods are provided for assessing the potency of blockade agents (e.g. antibodies) in inhibiting the PD-1/PD-L2 immune checkpoint pathway. Exemplary systems comprise: (1) a Jurkat cell displaying T Cell Receptor (TCR) complex and PD-1 on its surface and expressing an NFAT-promoter-induced luciferase reporter (e.g., luc2); (2) an artificial antigen presenting cell (aAPC) displaying anti-CD3 antibody and PD-L2 on its surface; and (3) a test blockade agent (e.g., antibody to PD-1 or PD-L2). In some embodiments, in the absence of the blockade agent (or in the event that the test blockade agent is ineffective), the PD-1 and PD-L2 interact and inhibit activation of the NFAT promoter by the anti-CD3-antibody-bound TCR complex; therefore, luciferase expression is inhibited, and the reporter signal is diminished. In the presence of a potent blockade agent, the interaction of the PD-1, and PD-L2 is inhibited, the NFAT promoter is activated, luciferase expression is enhanced, and the reporter signal increases. In some embodiments, when Jurkat effector cells and aAPCs are co-cultured, little or no luciferase is expressed, and little or no signal is generated (e.g., because the PD-1/PD-L2 complex inhibits reporter expression); however, addition of a potent blockade agent results in increased expression of luciferase and a gain of signal from the system.

In some embodiments, systems, and methods are provided for assessing the potency of blockade agents (e.g. antibodies) in inhibiting an immune checkpoint pathway dependent upon the interaction of CD80/CD86 and CTLA-4. Exemplary systems comprise: (1) a Jurkat cell displaying co-stimulatory receptor CD28, CTLA-4, the TCR complex, and comprising IL-2-promoter-dependent luciferase reporter construct; (2) an artificial antigen presenting cell (aAPC) displaying CD80/CD86 and anti-CD3 antibody Fab domain on its cell membrane; and (3) a test blockade agent (e.g., antibody to CTLA-4 or CD80/CD86). Upon the interaction of the aAPC and Jurkat effector cell, anti-CD3 Ab activates the TCR complex and CD80/CD86 co-stimulates CD28. This leads to activation of the IL-2 promoter, driving luciferase expression; however, CTLA-4 has higher binding affinity with CD80/CD86 than its binding affinity with CD28. It inhibits interaction of CD80/CD86 with CD28, and luciferase expression from the IL-2 promoter. In the presence of an inhibitor of CTLA-4 with CD80/CD86 interaction, the activated CD28 activates expression of the luciferase reporter from IL-2 promoter, and luciferase signal is elevated.

In some embodiments, systems, and methods are provided for assessing the potency of blockade agents (e.g. antibodies) in inhibiting the TIGIT/CD155 immune checkpoint pathway. Exemplary systems comprise: (1) a Jurkat T cell displaying co-stimulatory receptor CD226, TIGIT, the TCR complex, and comprising IL-2-promoter-dependent luciferase reporter construct; (2) an artificial antigen presenting cell (aAPC) displaying CD155 and anti-CD3 antibody Fab domain on its cell membrane; and (3) a test blockade agent (e.g., antibody to TIGIT or CD155). Upon the interaction of the aAPC and Jurkat effector cell, anti-CD3 Ab activates the TCR complex and CD155 co-stimulates CD226. These lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

In some embodiments, systems and methods are provided for assessing the potency of blockade agents (e.g. antibodies) in inhibiting the TIGIT/CD155 immune checkpoint pathway. Exemplary systems comprise: (1) a CEM T cell displaying co-stimulatory receptor CD226, TIGIT, the TCR complex, and comprising IL-2-promoter-dependent luciferase reporter construct (e.g., artificial CEM effector cell); (2) an artificial antigen presenting cell (aAPC) displaying CD155 and anti-CD3 antibody Fab domain on its cell membrane; and (3) a test blockade agent (e.g., antibody to TIGIT or CD155). Upon the interaction of the aAPC and CEM effector cell, anti-CD3 Ab activates the TCR complex and CD155 co-stimulates CD226. Both lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

In some embodiments, systems, and methods are provided for assessing the potency of blockade agents (e.g. antibodies) in inhibiting the PD-1/PD-L1 immune checkpoint pathway. Exemplary systems comprise: (1) a Jurkat T cell displaying PD-1, the TCR complex, and endogenous IL-2 gene driven by native IL-2 promoter; (2) an artificial antigen presenting cell (aAPC) displaying PD-L1 and anti-CD3 antibody Fab domain on its cell membrane; and (3) a test blockade agent (e.g., antibody to PD-1 or PD-L1). Upon the interaction of the aAPC and Jurkat effector cell, anti-CD3 Ab activates the TCR complex, activates IL-2 promoter, and increases IL-2 production; however, the interaction of PD-1 and PD-L1 inhibits activation of IL-2 expression from IL-2 promoter, and little production of IL-2 proteins occurs. In the presence of an inhibitor of the PD-1/PD-L1 interaction, the activated TCR complex activates IL-2 expression from IL-2 promoter, and IL-2 production in the media is elevated.

In some embodiments, systems and methods are provided for assessing the potency of blockade agents (e.g. antibodies) in inhibiting the LAG-3/MHC II immune checkpoint pathway. Exemplary systems comprise: (1) a Jurkat T cell displaying LAG-3, the TCR complex, and comprising NFAT-promoter-dependent luciferase reporter construct; (2) an artificial antigen presenting cell (aAPC) in a Raji B cell displaying MHC II on its cell membrane; and (3) a test blockade agent (e.g., antibody to LAG-3 or MHC II). Upon the interaction of the APC and Jurkat effector cell, superantigen presented by MHC II activates the TCR complex and activation of NFAT promoter driving luciferase expression; however, LAG-3 binding with MHC II inhibits TCR activation. This leads to inactivation of luciferase expression from the NFAT promoter. In the presence of an inhibitor of LAG-3/MHC II interaction, the activated TCR activates expression of the luciferase reporter from NFAT promoter, and the luciferase signal is elevated.

In some embodiments, systems comprise any suitable combination of the aAPCs, effector cells, ICLs, ICRs, promoters, reporters, cell types, etc. described herein.

In some embodiments, systems and methods described herein make use of the activation of transcription from the TCR activation pathway, downstream from proper engagement of the TCR by an APC (e.g., anti-CD3 antibody binding to the TCR complex). In some embodiments, effector cells are provided (e.g., engineered) with a reporter construct that signals activation of the cells. In some embodiments, a construct is provided with a reporter (e.g., luciferase) gene under the control of a TCR activation pathway. Upon activation of the T cell activation pathways, increased expression of the reporter (e.g., luciferase) occurs and cell activation can be detected and/or quantified based on the signal from the reporter. As noted above, it has been experimentally demonstrated that resting T-lymphocytes can be activated by anti-CD3 antibodies (Tsoukas et al, J Immunol. 1985 Sep;135(3):1719-23.; herein incorporated by reference in its entirety). Embodiments described herein are not limited to activation by anti-CD3. In some embodiments, other T cell stimulatory signals (e.g., displayed on aAPCs) are used to activate T cells, which is detected by a TCR activation pathway-dependent reporter. When effector cells comprising TCR activation pathway-dependent reporters are contacted by aAPCs displaying anti-CD3 antibodies (or fragments thereof), the T cell activation pathways are activated, and the reporter signal increases. However, upon formation of an inhibitory ICR/ICL complex (e.g., between an ICR on the effector cell and an ICL on the aAPC), T cell activation by the properly engaged TCR is inhibited, and transcription of the reporter from the TCR activation pathway is reduced. Blockade of the ICR/ICL complex by a blockade agent, restores TCR activation of T cell, and transcription of the reporter from the TCR activation pathway is increased.

In some embodiments, a TCR activation pathway-dependent reporter construct comprises: (1) a TCR activation pathway response element or promoter, and (2) a nucleic acid encoding a detectable reporter peptide, polypeptide or protein. In some embodiments, effector cells (e.g., Jurkat cells) are stably transfected with a TCR activation pathway-dependent reporter construct. Transfection is performed by methods well understood in the art; suitable methods include chemical-based transfection, non-chemical transfection, particle-based transfection, viral-based transfection, electroporation, and gene editing technology to monitor endogenous promoter activity or other known transfection methods.

An NFAT promoter is a nucleic acid sequence that contains one or more NFAT response elements. When bound by an NFAT transcription factor (e.g., NFATc1, NFATc2, NFATc3, NFATc4, or NFAT5), transcription of associated nucleic acid sequences (e.g., downstream genes) is enhanced. One suitable NFAT response element is:
GGAGGAAAAACTGTTTCATACAGAAGGCGT (SEQ ID NO: 1)

In some embodiments, an NFAT promoter comprises repeats of SEQ ID NO: 1. In some embodiments, an NFAT promoter comprises one or more NFAT response elements having at least 50% (e.g., >50%, >60%, >70%, >80%, >90%, >95%, and ranges therein) sequence identity to SEQ ID NO: 1, wherein the NFAT response elements bind to an NFAT family transcription factor to enhance transcription of associate (e.g., downstream) genes.

In some embodiments, the TCR activation pathway response element or promoter is an IL-2 promoter sequence, an NFkappaB response element that binds to an NFkappaB family transcription factor to enhance transcription of associated (e.g., downstream) genes, an AP1 response elements bind to an AP1 family transcription factor to enhance transcription of associated (e.g., downstream) genes, or any combination of the above response elements.

In some embodiments, the reporter of the TCR activation pathway-dependent reporter construct is any peptide, polypeptide, or protein that produces a detectable signal including, but not limited to, a luciferase, a beta lactamase, CAT, SEAP, a fluorescent protein, etc. In some embodiments, the reporter is a fluorescent or bioluminescent reporter. In certain embodiments, a bioluminescent reporter is a luciferase. In some embodiments, a luciferase is selected from those found in *Omphalotus olearius,* fireflies (e.g., *Photinini*)*, Renilla reniformis,* mutants thereof, portions thereof, variants thereof, and any other luciferase enzymes suitable for the systems and methods described herein. Embodiments described herein are not limited by the potential identity of the reporter.

In some embodiments, effector cells are provided that express PD-1 (e.g., for use in systems comprising aAPCs displaying PD-L1). In some embodiments, effector cells are provided that display PD-1 on their surface. In some embodiments, effector cells express and/or display a peptide/polypeptide with at least 50% sequence identity (e.g., >50%, >60%, >70%, >80%, >90%, >95%, or more) with all or a portion of PD-1, and retain at least 50% (e.g., >50%, >60%, >70%, >80%, >90%, >95%, or more) of the affinity of PD-1 for PD-L1. In some embodiments, effector cells are provided that display or express a peptide/polypeptide that has at least 50% (e.g., >50%, >60%, >70%, >80%, >90%, >95%, or more) of the affinity of PD-1 for PD-L1, and at least 50% e.g., >50%, >60%, >70%, >80%, >90%, >95%, or more) of the capacity of PD-1 to inhibit activation of T cells upon binding to PD-L1.

In some embodiments, effector cells are provided that express CD28, CTLA-4, CD226, TIGIT, etc. In some embodiments, effector cells express and/or display a peptide/polypeptide with at least 50% sequence identity (e.g., >50%, >60%, >70%, >80%, >90%, >95%, or more) with all or a portion of CD28, CTLA-4, CD226, TIGIT, etc., and retain at least 50% (e.g., >50%, >60%, >70%, >80%, >90%, >95%, or more) of the affinity of CD28, CTLA-4, CD226, TIGIT, etc.) to ligands thereof (e.g., CD80/CD86, CD155, etc.). In some embodiments, effector cells are provided that display or express a peptide/polypeptide that has at least 50% (e.g., >50%, >60%, >70%, >80%, >90%, >95%, or more) of the affinity of a receptor (e.g., CD28, CTLA-4, CD226, TIGIT, etc.) for a ligand thereof (e.g., CD80/CD86, CD155, etc.), and at least 50% (e.g., >50%, >60%, >70%, >80%, >90%, >95%, or more) of the capacity of the receptor to effect activation of T cells upon binding to the appropriate ligand.

In some embodiments, effector cells are provided that express and/or display one or more ICRs other than PD-1 (e.g., for use in systems comprising aAPCs displaying ICLs other than PD-L1). For example, in some embodiments, effector cells are provided that express and/or display one or more of CTLA-4, BTLA, etc. Embodiments described herein are not limited to the listed ICRs. Any ICR capable of interacting with an ICL to inhibit the activity of an effector cell (e.g., T cell) may find use in the embodiments described herein.

In some embodiments, systems provided herein comprise aAPCs and effector cells, wherein the aAPCs have the potential to activate the effector cells (e.g., via anti-CD3 of the aAPC binding to the TCR complex of the effector cell), thereby eliciting the expression of a detectable reporter (e.g., NFAT-promoter-dependent reporter); however, the interaction of an ICL (PD-L1) on the aAPC and an ICR (PD-1) on the effector cells inhibits effector-cell activation and reporter expression. In some embodiments, exposing the system to a blockade agent inhibits the ICR/ICL interaction, enhancing effector cell activation, and increasing reporter expression. In some embodiments, the potency of the blockade agent is directly proportional to reporter expression.

In certain embodiments, it is the interaction of PD-1 and PD-L1 that limits the activity of the effector cell and reporter expression. Therefore, blockade of the PD-1/PD-L1 interaction activates effector cells and increases reporter expression and reporter signal. PD-1/PD-L1 blockade can be accomplished by a variety of mechanisms including antibodies that bind PD-1 or PD-L1. Examples of PD-1 and PD-L1 blockers are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699; each of which is herein incorporated by reference in its entirety. In certain embodiments, the blockage agents are selected from anti-PD-L1 antibodies and/or similar binding proteins such as NIVOLUMAB (MDX 1106, BMS 936558, ONO 4538), a fully human IgG4 antibody that binds to and blocks the activation of PD-1 by its ligands PD-L1 and PD-L2; LAMBROLIZUMAB (MK-3475 or SCH 900475), a humanized monoclonal IgG4 antibody against PD-1; CT-011 a humanized antibody that binds PD-1; AMP-224 is a fusion protein of PD-L2 with an antibody Fc portion; BMS-936559 (MDX- 1105-01) for PD-L1 (B7-H1) blockade.

In some embodiments, a test blockade agent (e.g., an agent (e.g., antibody) suspected of inhibiting PD-1/PD-L1 complex formation or with unknown effect on PD-1/PD-L1 complex formation) is administered to a system described herein to test for the potency of the test agent on interrupting the PD-1/PD-L1 interaction. More generally, embodiments herein comprise a test blockade agent (e.g., an agent (e.g., antibody) suspected of inhibiting ICR/ICL complex formation or with unknown effect on ICR/ICL complex formation) is administered to a system described herein to test for the potency of the test agent on interrupting the ICR/ICL interaction.

In some embodiments, provided herein are systems and methods for screening test blockade agents for the ability to: (1) inhibit ICR/ICL interactions, (2) activate effector cells, and/or (3) promote transcription from NFAT promoters. In some embodiments, test blockade agent is a compound, peptide, protein, antibody, etc., that is suspected of inhibiting a particular ICR/ICL interaction (e.g., the interaction of PD-1 with PD-L1, PD-1 with PD-L2, CTLA-4 with CD80/CD86, TIGIT with CD155, etc.) or that has demonstrated such inhibiting in another assay. In other embodiments, a test blockade agent has unknown effects on ICR/ICL interactions (e.g., the interaction of PD-1 with PD-L1, PD-1 with PD-L2, CTLA-4 with CD80/CD86, TIGIT with CD155, etc.). Test blockade agents that have not been characterized for effect on ICR/ICL interactions and/or ability to induce effector cell activation may be tested alone or in a screen of multiple test blockade agents (e.g., a high throughput screen) using systems and methods described herein.

In some embodiments, systems and methods are provided for screening multiple test blockade agents of unknown or unconfirmed effect on ICR/ICL interactions (e.g., the interaction of PD-1 with PD-L1, PD-1 with PD-L2, CTLA-4 with CD80/CD86, TIGIT with CD155, etc.). In some embodiments, multiple test blockade agents (e.g., 2, 3, 4, 5, 6, 7, 8, 9, 10, 20, 30, 40, 50, 100, or more, or ranges therein) are introduced into a single blockade assay system described herein. In such embodiments, upon detection of an inhibitory effect on ICR/ICL interactions (e.g., the interaction of PD-1 with PD-L1, PD-1 with PD-L2, CTLA-4 with CD80/CD86, TIGIT with CD155, etc.), as indicated by an increase in reporter signal, individual test blockade agents or smaller groups of the agents will be subsequently tested in separate blockade assay systems (e.g., in separate reaction vessels). In other embodiments, multiple test blockade agents (e.g., 2, 3, 4, 5, 6, 7, 8, 9, 10, 20, 30, 40, 50, 100, 200, 500, 1000, 2000, 5000, 10,000, 20,000, 50,000, 100,000, or more, or ranges therein) are introduced into a parallel blockade assay system described herein. Such parallel assays may be run using systems, methods, devices, robotics, and other equipment known in the art to facilitate large-scale and/or high throughput screens. For example, aAPCs and effector cells are co-cultured in 96- or 384-well plates, and robotics are used to efficiently introduce a library of test blockade agents and to detect reporter signal.

In some embodiments, reagents, compositions, devices, equipment, etc. are provided (e.g., along with the assay components described herein (e.g., effector cells and aAPCs) for carrying out blockade assays. In some embodiments, reagents may include culture media, serum, nutrient supplements (e.g., sufficient for the co-culturing of aAPCs and effector cells), buffers, reporter substrate (e.g., luciferin, coelenterazine (or a derivative thereof), etc.), control blockade agents (e.g., positive controls known to inhibit ICR/ICL interactions (e.g., the interaction of PD-1 with PD-L1, PD-1 with PD-L2, CTLA-4 with CD80/CD86, TIGIT with CD155, etc.)), etc. In some embodiments, culture flasks and vesicles, microcentrifuge tubes, microplates including 96- and 384-well plates, readers, water bath, CO₂ incubator, single channel and multi-channel pipettes, etc., are provided. In some embodiments, robotics for high throughput assays (e.g., regent dispensing, sample organization, reporter detection, etc.), instruments for reporter detection (e.g., fluorometer, luminometer, spectrometer, Taqman, ELISA plates etc.), etc. are provided.

In some embodiments, all or a portion of the components for performing a blockade assay described herein are provided as a kit. The components may be in separate containers that are packaged together or separately for convenience or other considerations. In some embodiments, reagents (e.g., aAPCs, effector cells, etc.) are provided in predetermined amounts according to the size and/or number of blockade assays a user intends to perform. In some embodiments, accessories (e.g., vessels, plates, etc.), instructions, other reagents (e.g., buffers, controls, media, reporter substrate, etc.), etc., are also provided. In some embodiments, a user provides one or more components necessary for using the components (e.g., media, blockade agent(s), reporter substrate, etc.) of the kit to perform a blockade assay described herein.

In some embodiments, methods are provided utilizing the compositions and systems described herein. Methods utilizing the artificial antigen presenting cells (aAPCs) are provided, for example: to screen blockade agents, to develop and further modify and optimize blockade agents, to test the potency of blockade agents, to modulate immunity of effector cells, to identify ICRs and/ICLs, to test immune function in vitro or in vivo, etc. In some embodiments, methods utilize aAPCs in combination with artificial effector cells (e.g., comprising engineered receptors, comprising engineered antigen response elements, comprising engineered reporter constructs, etc.) or natural effector cells (e.g., effector cells from human or animal source comprising only intrinsic components). In some embodiments, methods are performed *in vitro, in vivo, in situ,* in a whole organism (e.g., human or animal model), in cell culture, etc. In some embodiments, methods provided herein utilize any of the aAPC, effector cell, blockade agent, or other components described in connection with the compositions and systems herein.

In some embodiments, methods comprise contacting (e.g., co-culturing): (a) an effector cell (e.g., natural or engineered) comprising an ICR, antigen responsive element (e.g., TCR complex), and a reporter (e.g., exogenous or intrinsic) with (b) an aAPC described herein (e.g., displaying an ICL and activator of an antigen responsive element (e.g., TCR activator). In some embodiments, methods further comprise detecting signal from said reporter to quantify: activation of effector cell immunity by the aAPC, modulation of effector cell activation by the formation of an ICR/ICL complex, etc. In some embodiments, methods further comprise contacting the aAPC and/or effector cell with a blockade agent or test blockade agent, and detecting signal from said reporter to quantify the effect of the blockade agent on one or more of: activation of effector cell immunity, formation of an ICR/ICL complex, modulation of effector cell activation by the ICR/ICL complex, an antigen-responsive-element-dependent pathway, an ICR/ICL-dependent pathway, etc.

In some embodiments, provided herein are methods comprising: (a) contacting: (i) an artificial antigen presenting cell (aAPC) displaying on its surface: (A) a T cell receptor (TCR) activator and (B) an immune checkpoint ligand (ICL) with (ii) an effector cell comprising a TCR complex and displaying on its surface an immune checkpoint receptor (ICR), wherein interaction of the TCR activator with the TCR complex activates a TCR signaling pathway, wherein said ICR and said ICL form an ICR/ICL complex upon interaction, and wherein formation of the ICR/ICL complex enhances or inhibits TCR activation and/or the TCR signaling pathway; and (b) detecting the presence, absence, and/or level of TCR activation in said effector cell. In some embodiments, methods further comprise: (c) contacting said effector cell and/or said aAPC with a blockade agent or test blockade agent. In some embodiments, methods further comprise: (d) detecting the effect of said blockade agent or test blockade agent on (i) ICR/ICL complex formation, (ii) TCR activation, and/or (iii) the TCR signaling pathway. In some embodiments, one or more of (i) ICR/ICL complex formation, (ii) TCR activation, and/or (iii) the TCR signaling pathway are detected in the presence and absence of said blockade agent or test blockade agent to determine an effect.

In some embodiments, provided herein are methods comprising: (a) contacting: (i) an artificial antigen presenting cell (aAPC) displaying on its surface: (A) a T cell receptor (TCR) activator, and (B) an immune checkpoint ligand (ICL), with (ii) an effector cell displaying on its surface an immune checkpoint receptor (ICR) and comprising a TCR complex and reporter (e.g., intrinsic or exogenous), wherein interaction of the TCR activator with the TCR complex activates a TCR signaling pathway, wherein said ICR and said ICL form an ICR/ICL complex upon interaction, wherein formation of the ICR/ICL complex enhances or inhibits TCR activation and/or the TCR signaling pathway, and wherein a detectable signal from said reporter (e.g., activity, luminescence, fluorescence, expression level, concentration, localization, protein-protein interaction, etc.) correlates with the level of TCR activation; and (b) detecting the presence, absence, and/or level of TCR activation in said effector cell. In some embodiments, methods further comprise: (c) contacting said effector cell and/or said aAPC with a blockade agent or test blockade agent. In some embodiments, methods further comprise: (d) detecting the effect of said blockade agent or test blockade agent on (i) ICR/ICL complex formation, (ii) TCR activation, and/or (iii) the TCR signaling pathway. In some embodiments, one or more of (i) ICR/ICL complex formation, (ii) TCR activation, and/or (iii) the TCR signaling pathway are detected in the presence and absence of said blockade agent or test blockade agent to determine an effect.

In some embodiments, aAPCs are introduced into a system (e.g., cell culture system, whole organism, tissue sample, blood sample, etc.), and the interaction of the aAPC and/or a blockade agent with effector cells within the system (or pathways downstream) are assayed (e.g., TCR/TCR-activator interaction, ICL/ICR interaction, disruption of the ICR/ICL interaction by the blockade agent, etc.). In some embodiments, the effector cells are primary cells (e.g., human or animal cells (e.g., isolated or derived from an organism or a cell culture line) with only intrinsic components), and the interaction is assayed based on, for example, a characteristic of an intrinsic reporter within the effector cell (e.g., expression from a TCR-pathway-dependent promoter, activity of a TCR pathway protein, TCR activation-dependent cellular changes, TCR dependent protein-protein interactions and protein movement, or detection of reporter gene regulation by any gene delivery method in transient or stable integration, etc.). In other embodiments, the effector cells are artificial effector cells and comprise a reporter construct for detecting the effects of the aAPC/effect interaction.

In certain embodiments, provided herein are methods of assaying the potency of a blockade agent (or test blockade agent) to inhibit the interaction of an ICR and ICL or signaling from an ICR/ICL complex. In such embodiments, a reporter or a signal from a reporter (e.g., natural or engineered) within the effector cell is monitored (e.g., in the presence, absence, and/or varying concentration of blockade agent) to determine and/or quantify the potency of the blockade agent.

In some embodiments, methods of screening (e.g., a library of compounds, peptides, antibodies, etc.) test blockade agents are provided. In such embodiments, a number of substantially identical systems described herein are assayed in parallel to identify the response of the system to a number of test blockade agents. Such assays may be performed in low- or high- throughput formats and may be performed manually or may be automated.

In some embodiment, methods are provided in which the aAPCs described herein are introduced into a biological system (e.g., biological sample (e.g., tissue sample, blood sample, etc.), whole organism, cell culture system, etc.). In some embodiments, the biological system comprises natural effector cells and/or effector cells that have been engineered according to embodiments described herein.

In some embodiments, assays are performed as a service. In such embodiments, a user submits one or more test blockade agents to be tested. One or more blockade assays are performed according to the embodiments described herein, and the results (e.g., inhibitory potency of the test blockade agent) are reported to the user. Test results may be reported in any suitable format and may include raw data (e.g., reporter signal as a function of blockade agent concentration) in an analyzed form to provide the user with readily interpretable results.

Compositions, systems, and methods are described herein which comprise or utilize aAPCs. In some embodiments, such compositions, systems, and methods may instead comprise phospholipid droplets in place of the aAPCs. Such phospholipid droplets display and comprise the same elements as the aAPCs (e.g., TCR activator, immune checkpoint ligand, etc.) described herein and find use in the same methods (e.g., interacting with effector cells).

### EXPERIMENTAL

### Example 1

### PD-1/PD-Ll Blockade Assay Specificity

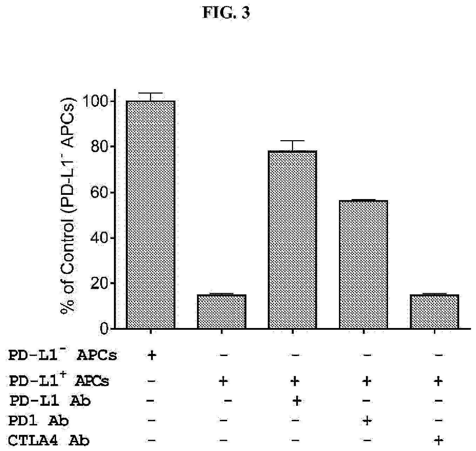

Experiments were conducted during development of embodiments described herein to test the detectable response of the blockade assay depicted in Figure 1 to various conditions. Jurkat effector cells expressing PD-1 and NFAT-RE-luciferase reporter were co-cultured with PD-L1⁻ or PD-L1⁺ aAPC which both display anti-CD3 antibodies (Figure 2 and Figure 3). Luciferase activity significantly decreased in the co-culture system comprising Jurkat effector cells and PD-L1⁺ aAPC compared with co-culture system comprising Jurkat effector cells and PD-L1⁻ aAPC. Different blockage agents were incubated and compared in the system comprising Jurkat effector cells and PD-L1⁺ aAPCs (Figure 3). Only in the presence of specific blockage agent known to specifically blocking the PD-1/PD-L1 interaction, anti-PD-L1 antibody or anti-PD-1 antibody, but not in the presence of anti-CTLA-4 antibody, anti-CD3 antibody activated TCR complex and increased the expression of the luciferase reporter driven by the NFAT response element. Experiments demonstrated the specificity of the system to the various conditions tested.

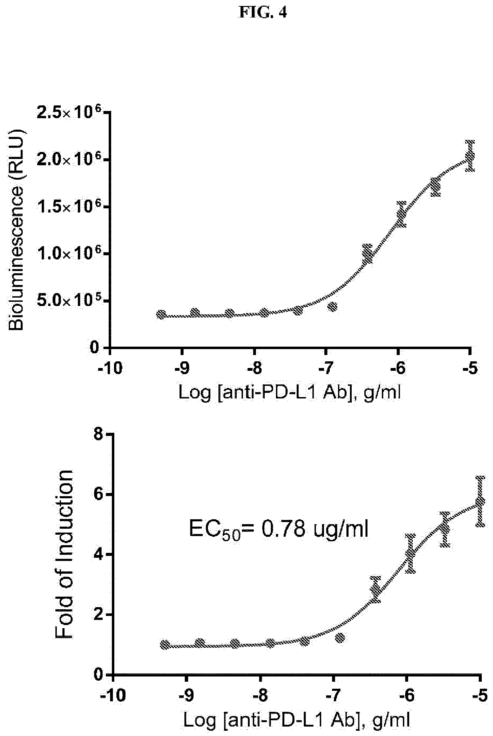

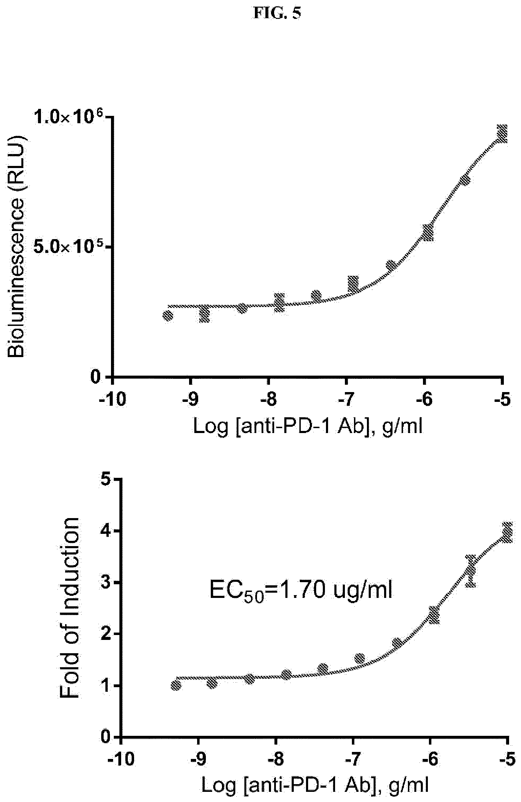

Experiments demonstrated that anti-PD-L1 antibody (Figure 4) and anti-PD-1 antibody (Figure 5) activity can be measured by the reporter system. Jurkat effector cells expressing PD-1 and NFAT-RE-luciferase reporter were co-cultured with anti-CD3/PD-L1⁺ aAPC, in the presence of serial dilution of anti-PD-L1 antibody (Figure 4) or anti-PD-1 antibody (Figure 5). Both blockage agents provided concentration-dependent inhibition of the PD-1/PD-L1 complex formation, resulting in concentration-dependent increase in luminescence signals and increased luciferase activity by 4-6 fold from the reporter system.

### Example 2

### PD-1/PD-L1 Blockade Assay using aAPCs not expressing anti-CD3

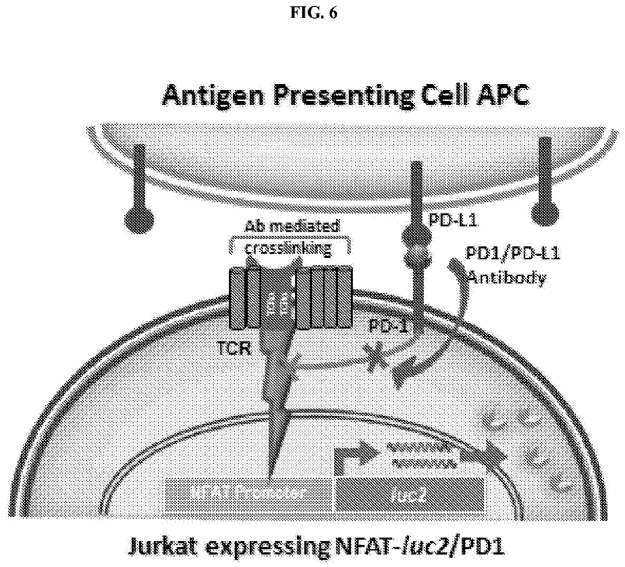

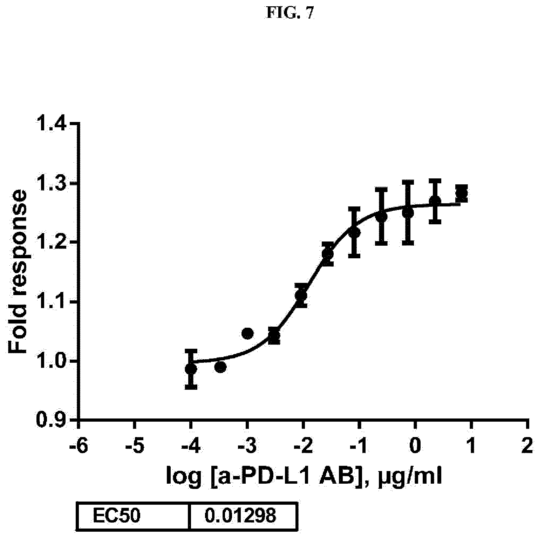

Experiments conducted during development of embodiments described herein demonstrate that blockade assay systems using aAPCs expressing PD-L1, but not expressing anti-CD3, in the presence of soluble anti-CD3 antibody (Figure 6). Jurkat effector cells expressing PD-1 and NFAT-RE-luciferase reporter were co-cultured with anti-CD3⁻/PD-L1⁺ aAPC, in the presence of soluble anti-CD3 antibody and serial dilution of blockage agent, anti-PD-L1 antibody (Figure 6). Blockage agent provided significantly reduced induction (< 1.3 fold) of NFAT-RE-dependent expression of luciferase (Figure 7), compared with 6 fold of increase of luciferase expression from same blockage agent in Figure 4.

### Example 3

### Anti-CD3/PD-L1 beads in place of anti-CD3/PD-L1 aAPCs in Blockade Assay

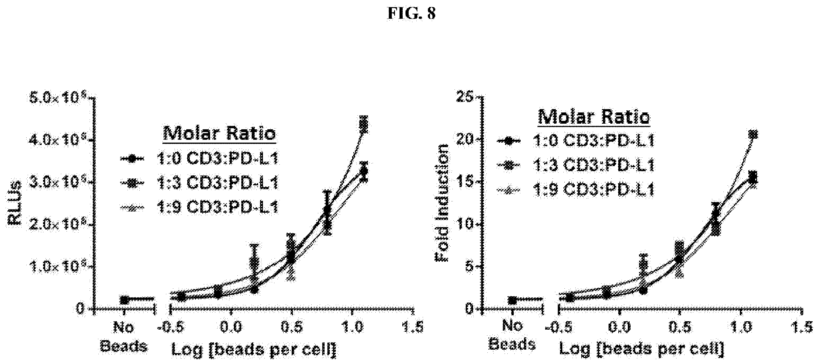

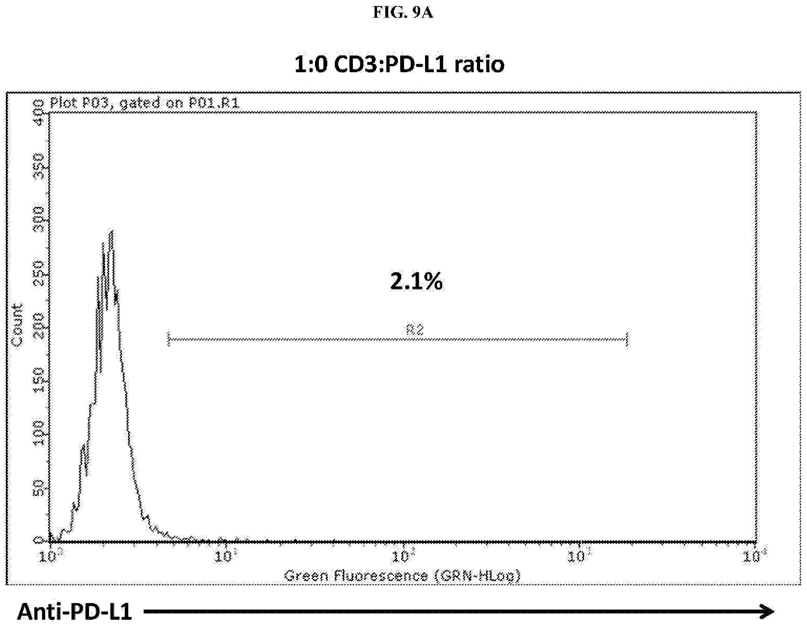

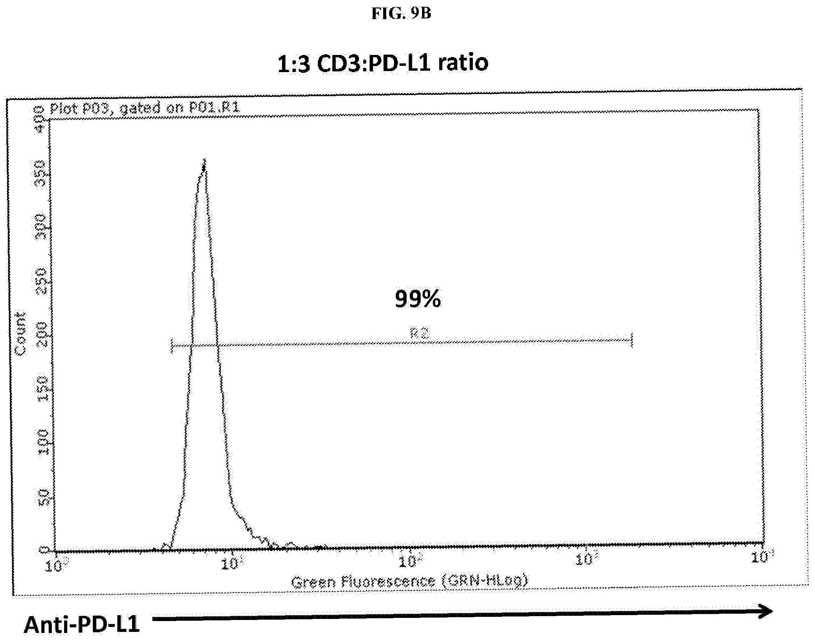

Experiments conducted during development of embodiments described herein demonstrate that anti-CD3/PD-L1 aAPC beads fail to inhibit activation of NFAT-promoter-dependent expression of luciferase by Jurkat effector cells (Figure 8). Beads were coated with a fixed amount of anti-CD3 (10% of protein). PD-L1-Fc chimera was simultaneously coated at 1:0 (no PD-L1), 1:3, and 1:9 molar ratios of anti-CD3:PD-L1-Fc. An irrelevant IgG was used to balance the molar ratios. Jurkat effector cells expressing PD-1 and NFAT-RE-luciferase were incubated with serial bead dilutions. NFAT-RE-dependent luciferase expression was similar whether 1:0, 1:3, or 1:9 anti-CD3:PD-L1-Fc beads were used. FACS analysis confirms the presence of PD-L1 on the beads (Figure 9). Beads described for Figure 8 were labeled with anti-PD-L1-FITC and analyzed by flow cytometry.

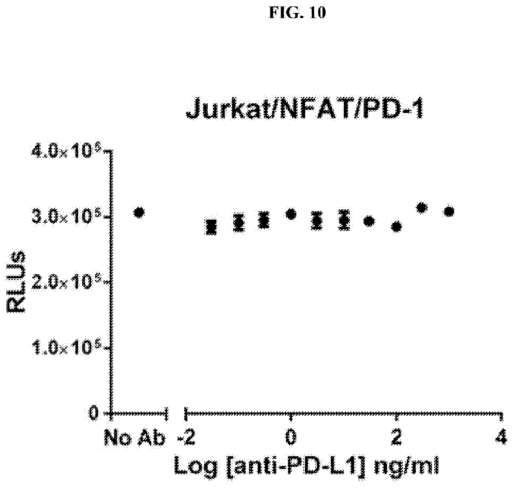

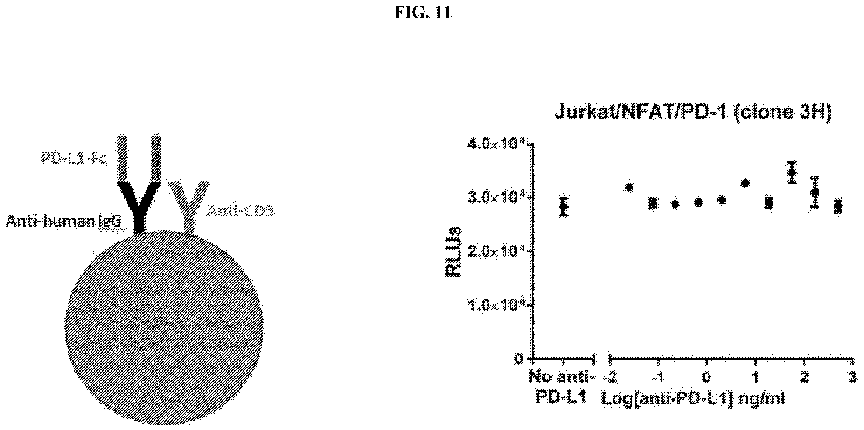

Anti-CD3/PD-L1 beads (1:9 molar ratio) failed to show PD-1/PD-L1 engagement induced inhibition of T cell activation, and anti-PD-L1 antibody failed to induced T cell activation (Figure 10). Jurkat effector cells expressing PD-1 and NFAT-RE-luciferase were treated with anti-CD3:PD-L1-Fc beads in the presence or absence of PD-L1 blocking antibody. NFAT-RE-dependent luciferase expression was unchanged in the presence of PD-L1 blocking antibody. Extended length of PD-L1 on the beads did not remedy the lack of activation by the beads (Figure 11). Shown is an illustration of the strategy to extend PD-L1 away from the bead. Beads were coated with anti-CD3:anti-human IgG at a 1:9 molar ratio. Beads were then incubated with PD-L1-Fc chimera (containing a human IgG1 Fc region fused to PD-L1) to load PD-L1 on the beads. Jurkat effector cells expressing PD-1 and NFAT-RE-dependent luciferase were treated with these beads in the presence or absence of PD-L1 blocking antibody. NFAT-RE-dependent luciferase expression was not affected by the presence of PD-L1 blocking antibody.

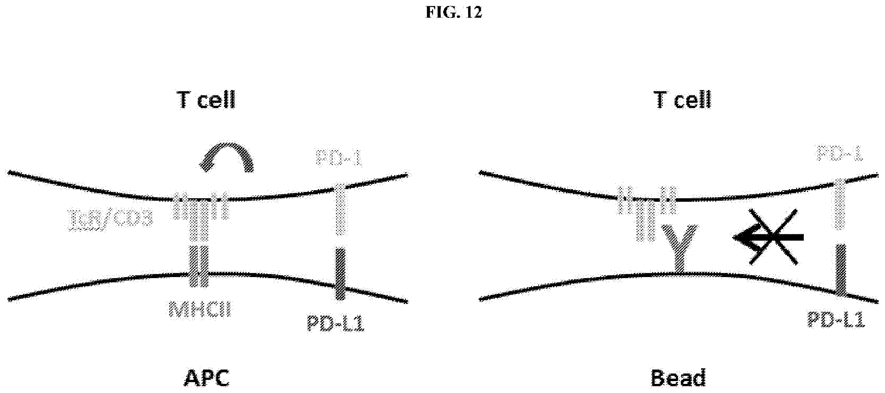

It is contemplated that the rigidity of the placement of anti-CD3 and PD-L1 on the beads may prevent effective T cell inhibition (Figure 12). Specifically, formation of an immune synapse between T cells and aAPCs requires the dynamic lateral localization of membrane-bound receptors and ligands. Upon binding PD-L1, PD-1 is known to be co-localized with the TCR in the immune synapse, where PD-1 can directly inhibit TCR complex signaling. When PD-L1 is localized in the plasma membrane of aAPCs, such lateral localization of PD-1/PD-L1 can be achieved (illustration on the left). When PD-L1 is bound on a bead surface, the position of PD-L1 is static, and lateral movement is restricted (illustration on the right). Thus, PD-1/PD-L1 will not be co-localized with the TCR, and PD-1 will not be able to inhibit TCR complex signaling. This is consistent with published research indicating that PD-1 forms negative co-stimulatory microclusters with the TCR complex, which are required to inhibit proximal TCR signaling (Yokosuka et al. J Exp Med. 2012 Jun 4;209(6): 1201-17.; herein incorporated by reference in its entirety). The embodiments described herein are not limited to any particular mechanism of action and an understanding of the mechanism of action is not necessary to practice such embodiments.

### Example 4

### PD-1/PD-L2 Blockade Assay Specificity

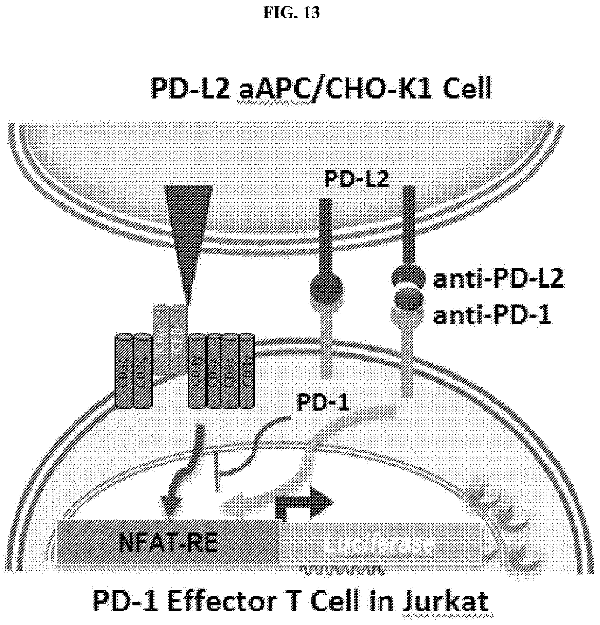

Experiments were conducted during development of embodiments described herein to test the detectable response of the blockade assay depicted in Figure 13 to various conditions. An aAPC displaying PD-L2 and anti-CD3 antibody Fab domain on its cell membrane is shown interacting with a Jurkat T cell displaying PD-1, the TCR complex, and comprising NFAT-promoter-dependent luciferase reporter construct (Jurkat effector cell). Upon the interaction of the aAPC and Jurkat effector cell, anti-CD3 Ab activates the TCR complex and NFAT-promoter; however, the interaction of PD-1 and PD-L2 inhibits TCR activation and luciferase expression from the NFAT promoter, and little expression of the luciferase occurs. In the presence of an inhibitor of the PD-1/PD-L2 interaction, the activated TCR complex activates expression of the luciferase reporter from the NFAT promoter, and luciferase signal is elevated.

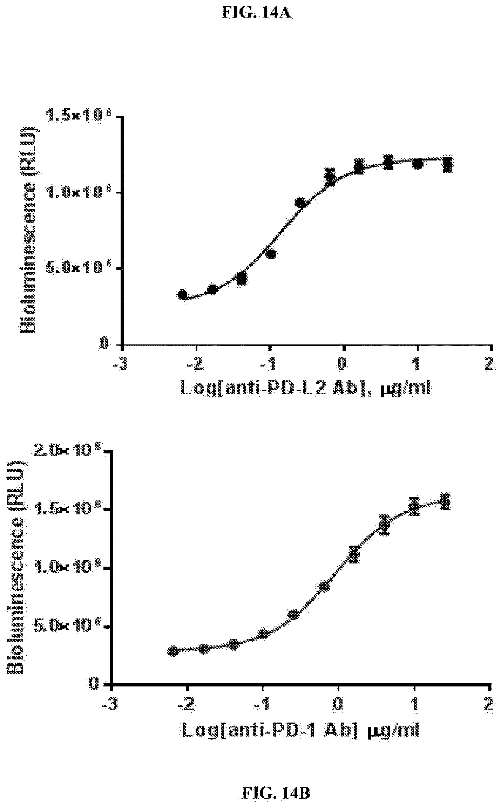

The experiments demonstrated that anti-PD-1 antibody (Figure 14b) and anti-PD-L2 antibody (Figure 14a) activity were measured by the reporter system. Jurkat effector cells expressing PD-1 and NFAT-RE-luciferase reporter were co-cultured with anti-CD3/PD-L2⁺ aAPC, in the presence of serial dilution of anti-PD-L2 antibody (Figure 14a) or anti-PD-1 antibody (Figure 14b). Both blockage agents provided concentration-dependent activation of reporter expression, resulting in concentration-dependent increase in luminescence signals and increased luciferase activity.

### Example 5

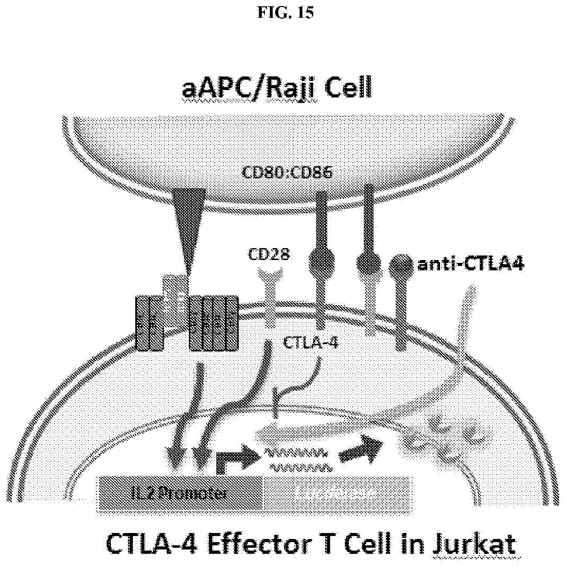

Experiments were conducted during development of embodiments described herein to assess inhibitors of immune checkpoint receptor CTLA-4 and its ligand, CD80/CD86, as depicted in Figure 15. An aAPC in Raji B cells displaying CD80/CD86 and anti-CD3 antibody Fab domain on its cell membrane is shown interacting with a Jurkat T cell displaying co-stimulatory receptor CD28, CTLA-4, the TCR complex, and comprising IL-2-promoter-dependent luciferase reporter construct (Jurkat effector cell). Upon the interaction of the aAPC and Jurkat effector cell, anti-CD3 Ab activates the TCR complex and CD80/CD86 co-stimulates CD28. This leads to activation of the IL-2 promoter, driving luciferase expression; however, CTLA-4 has higher binding affinity with CD80/CD86 than its binding affinity with CD28. It inhibits interaction of CD80/CD86 with CD28 and luciferase expression from the IL-2 promoter. In the presence of an inhibitor of CTLA-4 with CD80/CD86 interaction, the activated CD28 activates expression of the luciferase reporter from IL-2 promoter, and luciferase signal is elevated.

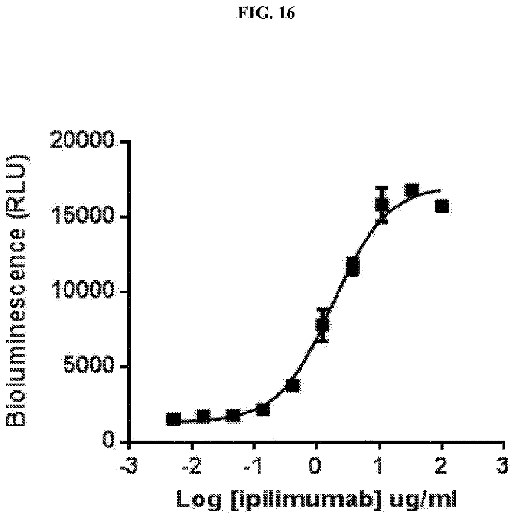

The experiments demonstrated measurement of activity of the anti-CTLA-4 antibody, ipilimumab, by the reporter system (Figure 16). Jurkat effector cells expressing CTLA-4 and an IL-2-luciferase reporter were co-cultured with anti-CD3/CD80/CD86 ⁺ aAPC, in the presence of a serial dilution of ipilimumab. The blockade agent provided concentration-dependent activation of reporter expression resulting in concentration-dependent increase in luminescence signals and increased luciferase activity.

### Example 6

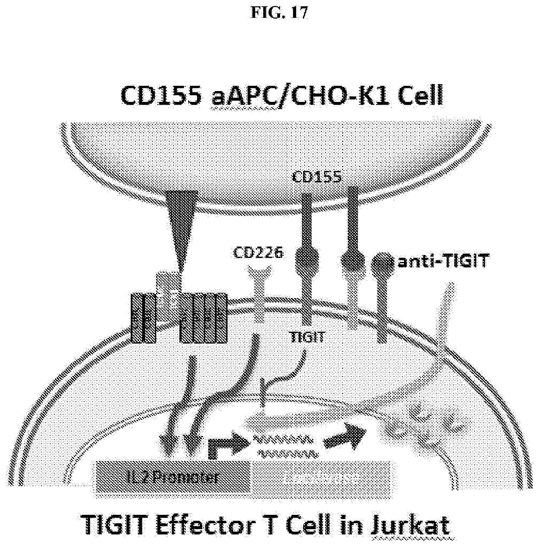

Experiments were conducted during development of embodiments described herein for assessing inhibitors of immune checkpoint receptor TIGIT and its ligand, CD155, as depicted in Figure 17. An aAPC displaying CD155 and anti-CD3 antibody Fab domain on its cell membrane is shown interacting with a Jurkat T cell displaying co-stimulatory receptor CD226, TIGIT, the TCR complex, and comprising IL-2-promoter-dependent luciferase reporter construct. Upon the interaction of the aAPC and Jurkat effector cell, anti-CD3 Ab activates the TCR complex and CD155 co-stimulates CD226. These lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

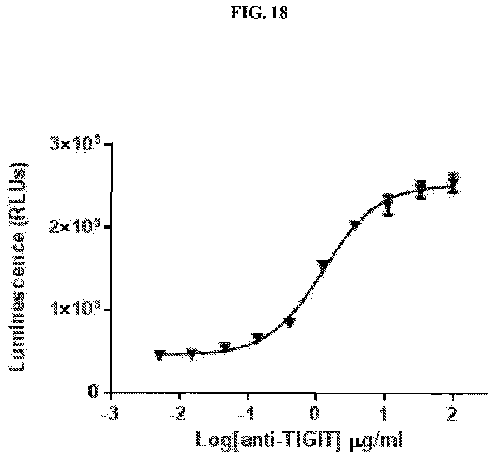

The experiments demonstrated that anti-TIGIT antibody (Figure 18) activity was measured by the reporter system. Jurkat effector cells expressing TIGIT and an IL-2 promoter dependent-luciferase reporter were co-cultured with anti-CD3/CD155 ⁺ aAPC, in the presence of a serial dilution of anti-TIGIT antibody. The blockage agent provided concentration-dependent activation of reporter expression by an anti-TIGIT antibody resulting in an increase in luminescence signals and increased luciferase activity.

### Example 7

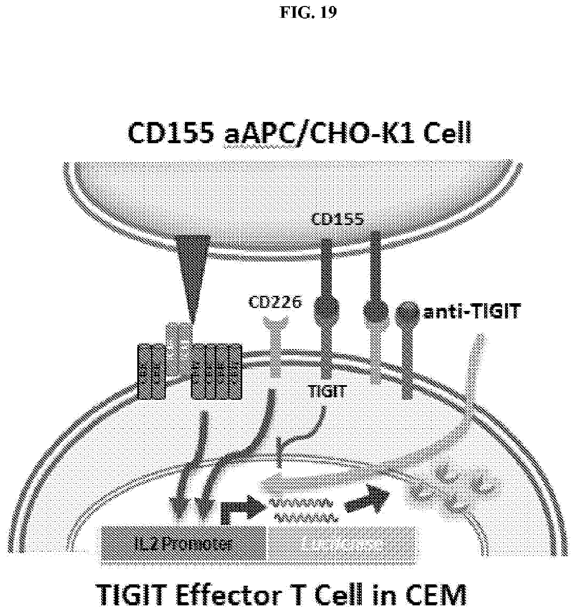

Experiments were conducted during development of embodiments described herein for assessing inhibitors of immune checkpoint receptor TIGIT and its ligand, CD155, as depicted in Figure 19. An aAPC displaying CD155 and anti-CD3 antibody Fab domain on its cell membrane is shown interacting with a CEM T cell displaying co-stimulatory receptor CD226, TIGIT, the TCR complex, and comprising IL-2-promoter-dependent luciferase reporter construct (CEM effector cell). Upon the interaction of the aAPC and CEM effector cell, anti-CD3 Ab activates the TCR complex and CD155 co-stimulates CD226. Both lead to activation of IL-2 promoter driving luciferase expression; however, CD155 has higher binding affinity with TIGIT than with CD226. Therefore, TIGIT inhibits interaction of CD155 with CD226 and the activation of luciferase expression from the IL-2 promoter. In the presence of an inhibitor of the TIGIT/CD155 interaction, the activated CD226 activates expression of the luciferase reporter from IL-2 promoter, and luciferase signal is elevated.

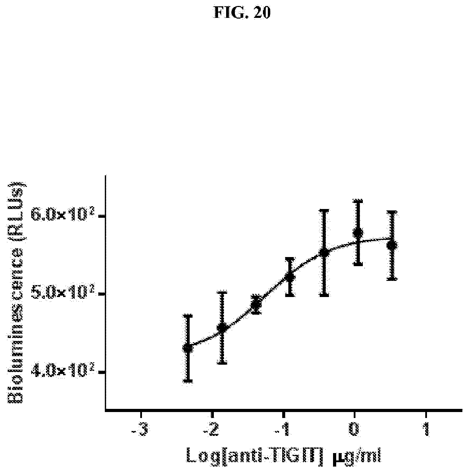

The experiments demonstrated that anti-TIGIT antibody (Figure 20) activity was measured by the reporter system. CEM T effector cells expressing TIGIT and an IL-2 promoter dependent-luciferase reporter were co-cultured with anti-CD3/CD155 ⁺ aAPC, in the presence of a serial dilution of anti-TIGIT antibody. The blockage agent provided concentration-dependent activation of reporter expression by an anti-TIGIT antibody resulting in an increase in luminescence signals and increased luciferase activity.

### Example 8

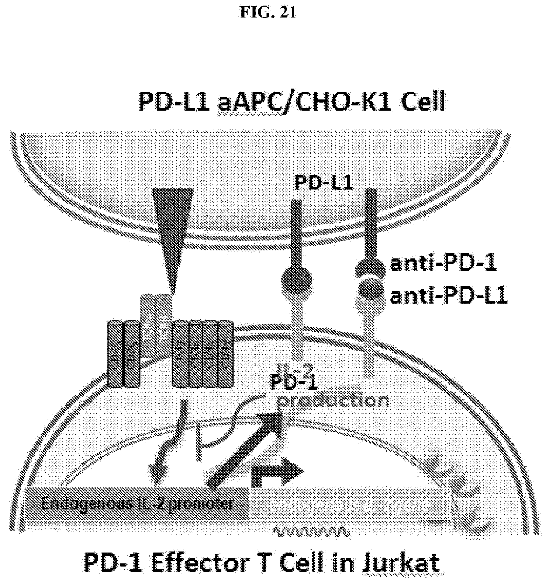

Experiments were conducted during development of embodiments described herein for assessing inhibitors of immune checkpoint receptor PD-1 and its ligand PD-L1, as depicted in Figure 21. An aAPC displaying PD-L1 and anti-CD3 antibody Fab domain on its cell membrane is shown interacting with a Jurkat T cell displaying PD-1, the TCR complex, and endogenous IL-2 gene driven by native IL-2 promoter. Upon the interaction of the aAPC and Jurkat effector cell, anti-CD3 Ab activates the TCR complex, activates IL-2 promoter and increases IL-2 production; however, the interaction of PD-1 and PD-L1 inhibits activation of IL-2 expression from IL-2 promoter, and little production of IL-2 proteins occurs. In the presence of an inhibitor of the PD-1/PD-L1 interaction, the activated TCR complex activates IL-2 expression from IL-2 promoter, and IL-2 production in the media is elevated.

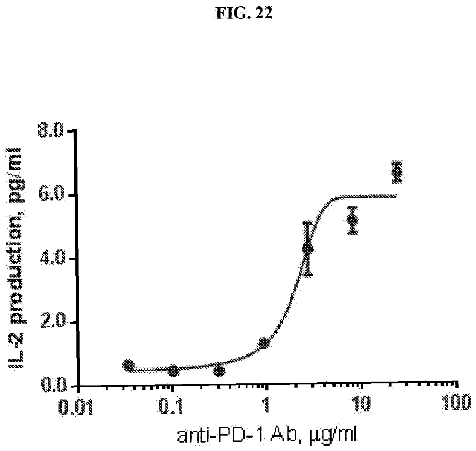

The experiments demonstrated that anti-PD-1 antibody (Figure 22) activity was measured by ELISA. Jurkat T effector cells expressing PD-1 and an endogenous IL-2 gene driven by native IL-2 promoter were co-cultured with anti-CD3/PD-L1 ⁺ aAPC, in the presence of a serial dilution of anti-PD-1 antibody. The blockage agent provided concentration-dependent IL-2 production by an anti-PD-1 antibody.

### Example 9

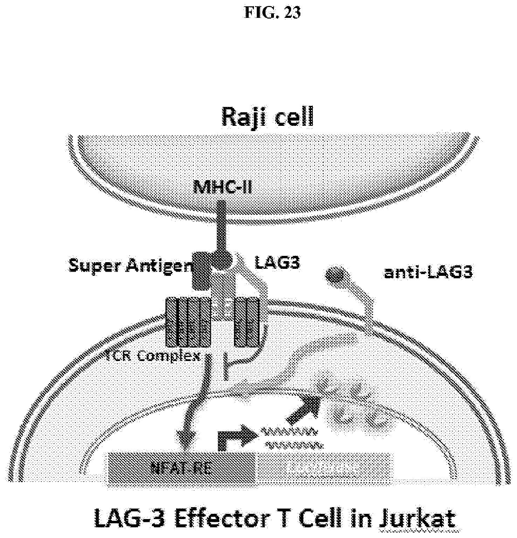

Experiments were conducted during development of embodiments described herein for assessing inhibitors of immune checkpoint receptor LAG-3 and its ligand, MHC II, as depicted in Figure 23. An APC in Raji B cells displaying MHC II on its cell membrane is shown interacting with a Jurkat T cell displaying LAG-3, the TCR complex, and comprising NFAT-promoter-dependent luciferase reporter construct (Jurkat effector cells). Upon the interaction of the APC and Jurkat effector cell, super antigen presented by MHC II activates the TCR complex and activation of NFAT promoter driving luciferase expression; however, LAG-3 binding with MHC II inhibits TCR activation. This leads to inactivation of luciferase expression from the NFAT promoter. In the presence of an inhibitor of LAG-3/MHC II interaction, the activated TCR activates expression of the luciferase reporter from NFAT promoter, and luciferase signal is elevated.

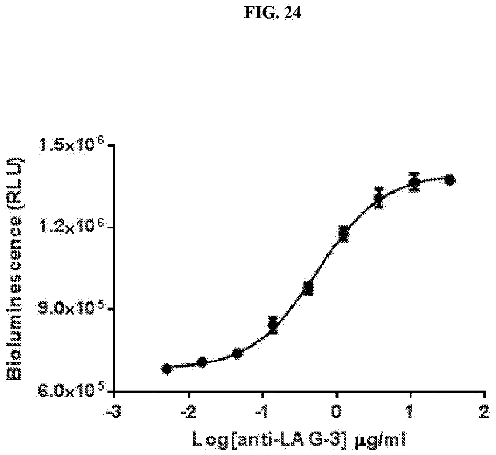

The experiments demonstrated that anti-LAG-3 antibody (Figure 24) activity was measured by the reporter system. Jurkat effector cells expressing LAG-3 and NFAT RE-luciferase reporter were co-cultured with anti-MHC II ⁺ aAPC, in the presence of a serial dilution of anti-LAG-3 antibody. The blockage agent provided concentration-dependent activation of reporter expression by an anti-LAG-3 antibody resulting in an increase in luminescence signals and increased luciferase activity.

### Example 10

Experiments were conducted during development of embodiments described herein for assessing inhibitors of immune checkpoint receptors TIGIT and PD-1 and their ligands, CD155 and PD-L1, respectively, as shown in Figure 25. An aAPC displaying PD-L1, CD155, and anti-CD3 antibody Fab domain on its cell membrane is shown interacting with a Jurkat T cell displaying PD-1, co-stimulatory receptor CD226, TIGIT, the TCR complex, and comprising IL2-promoter-dependent luciferase reporter construct. Upon the interaction of the aAPC and Jurkat effector cell, anti-CD3 Ab activates the TCR complex and CD155 co-stimulates CD226. These led to activation of IL-2 promoter driving luciferase expression; however, the interaction of PD-1 with PD-L1 leads to reduced TCR pathway activation and reduced IL-2 promoter-dependent reporter expression. In addition, CD155 has higher binding affinity with TIGIT than with CD226. Therefore, TIGIT inhibits interaction of CD155 with CD226 and leads to reduced reporter expression. In the presence of an inhibitor of the PD-1/PD-L1 and/or TIGIT/CD155 interaction, the expression of the luciferase reporter from IL-2 promoter is enhanced, and the luciferase signal is elevated.

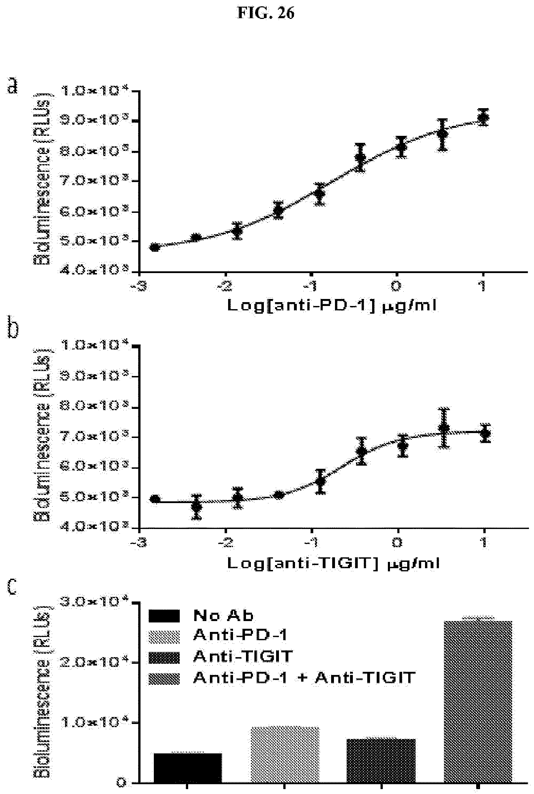

Figure 26a-b shows graphs depicting concentration-dependent activation of reporter expression by (a) an anti-PD-1 antibody and (b) an anti-TIGIT antibody in an exemplary PD-1/TIGIT blockade assay using a PD-1/TIGIT effector Cells in Jurkat-T cells and PD-L1/CD155 aAPC/CHO-K1 cells. Figure 26c shows the effects of a combination of anti-PD-1 and anti-TIGIT antibodies compared with either antibody alone.

All publications and patents provided herein incorporated by reference in their entireties. Various modifications and variations of the described compositions and methods of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the present invention.

Embodiments of the invention will now be described in the following numbered paragraphs:
1. A composition comprising an artificial antigen presenting cell (aAPC) or phospholipid droplet displaying on its surface a T cell receptor (TCR) activator and an immune checkpoint ligand (ICL).
2. The composition of paragraph 1, wherein upon interaction of the aAPC with an effector cell comprising a TCR and an immune checkpoint receptor (ICR), the TCR activator activates the TCR of the effector cell, the ICL interacts with the ICR, and forms an ICR/ICL complex.
3. The composition of paragraph 2, wherein formation of the ICR/ICL complex modulates the downstream effects of TCR activation.
4. The composition of paragraph 1, wherein the TCR activator is an anti-cluster of differentiation 3 (CD3) antibody, or an antibody fragment thereof, or major histocompatibility complex (MHC).
5. The composition of paragraph 1, wherein the ICL is selected from the group consisting of PD-L1, PD-L2, B7-H4, CD155, galectin-9, and HVEM.
6. The composition of paragraph 1, wherein the aAPC further comprises phospholipids, TCR activator, ICL, or immuno-stimulatory receptor ligand.
7. A system comprising:
   (a) an effector cell displaying on its surface an immune checkpoint receptor (ICR), and comprising a T cell receptor (TCR); and
   (b) an artificial antigen presenting cell (aAPC) or phospholipid droplet displaying on its surface a T cell receptor (TCR) activator and an immune checkpoint ligand (ICL);
   wherein the ICR and ICL form an ICR/ICL complex upon interaction.
8. The system of paragraph 7, wherein formation of the ICR/ICL complex results in modulation of TCR activation by the TCR activator and/or modulation of one or more TCR-dependent pathways.
9. The system of paragraph 8, wherein modulation comprises:
   (a) inhibition of TCR activation by the TCR activator and/or one or more TCR-dependent pathways; or
   (b) enhancement of TCR activation by the TCR activator and/or one or more TCR-dependent pathways.
10. The system of paragraph 7, wherein the TCR activator is an anti-cluster-of-differentiation-3 (CD3) antibody, or an antibody fragment thereof, or major histocompatibility complex (MHC).
11. The system of paragraph 7, wherein the ICL is selected from the group consisting of CD80/86 PD-L1, PD-L2, B7-H4, CD155, galectin-9, and HVEM.
12. The system of paragraph 7, wherein the ICR is selected from the group consisting of PD-1, CTLA-4, LAG-3, TIM-3, CD160, TIGIT, IL-10 receptor, and BTLA.
13. The system of paragraph 7, wherein the effector cell is selected from T cells including Jurkat, HuT-78, CEM, Molt-4 and primary T cells.
14. The system of paragraph 7, wherein the effector cell further comprises a reporter of: TCR activation, TCR pathway activation, and/or ICR/ICL complex modulation of TCR activation or TCR pathway activation.
15. The system of paragraph 14, wherein the reporter characteristic comprises: cellular concentration, expression, activity, localization, protein modification, or protein-protein interactions.
16. The system of paragraph 15, wherein formation of the ICR/ICL complex modulates said characteristic.
17. The system of paragraph 14, wherein the reporter is a natural reporter, intrinsic to the effector cell type, having a characteristic that is detectable and correlates to TCR activation, TCR pathway activation, and/or ICR/ICL complex modulation of TCR activation or TCR pathway activation.
18. The system of paragraph 14, wherein the reporter is an artificial reporter, exogenous to the effector cell type, having a characteristic that is detectable and correlates to TCR activation, TCR pathway activation, and/or ICR/ICL complex modulation of TCR activation or TCR pathway activation.
19. The system of paragraph 18, wherein the reporter is a gene, the expression of which is under the control of TCR-pathway-dependent reporter.
20. The system of paragraph 19, wherein the TCR-pathway-dependent reporter is a nuclear factor of activated T cells (NFAT) promoter.
21. The system of paragraph 18, wherein the reporter comprises a gene coding for a protein selected from the group consisting of a luciferase, a beta lactamase, CAT, SEAP, a fluorescent protein, and a quantifiable gene product.
22. The system of paragraph 7, further comprising a blockade agent or test blockade agent.
23. The system of paragraph 22, wherein the blockade agent inhibits formation of the ICR/ICL complex, resulting in modulation of TCR activation or TCR pathway activation.
24. The system of paragraph 22, wherein the blockade agent or test blockade agent is a small molecule, peptide, protein, or antibody.
25. A system comprising:
   (a) an effector cell comprising TCR and PD-1 on its surface, and an NFAT-promoter-dependent luciferase; and
   (b) an artificial antigen presenting cell (aAPC) displaying on its surface: anti-CD3 antibody or antibody fragment, and PD-L1.
26. The system of paragraph 25, wherein formation of a PD-1/PD-L1 complex results in a suppressed expression of the NFAT-promoter-dependent luciferase.
27. The system of paragraph 26, further comprising a blockade agent or test blockade agent.
28. The system of paragraph 27, wherein the blockade agent or test blockade agent inhibits formation of the PD-1/PD-L1 complex, resulting in an increased expression of the NFAT-promoter-dependent luciferase.
29. A method comprising:
   (a) forming a system comprising:
      (i) an effector cell displaying on its surface an immune checkpoint receptor (ICR), and comprising a T Cell Receptor (TCR) and a TCR-pathway-dependent reporter, and
      (ii) an artificial antigen presenting cell (aAPC) displaying on its surface a TCR activator and an immune checkpoint ligand (ICL); wherein the ICR and ICL form an ICR/ICL complex upon interaction, and wherein formation of the ICR/ICL complex results in modulation of TCR activation by the TCR activator and/or modulation of one or more TCR-dependent pathways; and
   (b) detecting said TCR-pathway-dependent reporter or a signal from said reporter.
30. The method of paragraph 29, further comprising adding to the system a blockade agent, wherein the blockade agent inhibits formation of the ICR/ICL complex or inhibits ICR/ICL-dependent modulation of TCR activation by the TCR activator and/or modulation of one or more TCR-dependent pathways.
31. The method of paragraph 30, wherein said TCR-pathway-dependent reporter or a signal from said reporter is detected: (1) before, (2) concurrent with, and/or (3) after addition of said blockade agent.
32. The method of paragraph 31, further comprising a step of comparing signal from (1) before, (2) concurrent with, and/or (3) after addition of said blockade agent to determine the effect of the blockade agent.
33. A method comprising:
   (a) co-culturing:
      (i) an effector cell displaying T cell Receptor (TCR) and an immune checkpoint receptor (ICR) on its surface, and TCR-pathway-dependent reporter, and
      (ii) an artificial antigen presenting cell (aAPC) displaying a TCR activator and an immune checkpoint ligand (ICL), wherein the ICR and ICL form an ICR/ICL complex upon interaction;
   (b) adding said test blockade agent;
   (c) detecting said signal from said reporter; and
   (d) comparing said signal from said reporter with a control, wherein a gain of signal with respect to the control indicates inhibition of said ICR/ICL complex by said test blockade agent.
34. A method of identifying an ICR/ICL blockade agent, comprising:
   (a) co-culturing:
      (i) an effector cell displaying T cell Receptor (TCR) and an immune checkpoint receptor (ICR) on its surface, and TCR-pathway-dependent reporter, and
      (ii) an artificial antigen presenting cell (aAPC) displaying anti-cluster-of-differentiation-3 (CD3) antibody and an immune checkpoint ligand, wherein the ICR and ICL form an ICR/ICL complex upon interaction;
   (b) detecting a signal from said reporter;
   (c) adding said test blockade agent;
   (d) repeating detection of said signal from said reporter; and
   (e) comparing said signal from step (b) with said signal from step (d), wherein a gain of signal from step (b) to step (d) indicates inhibition of said ICR/ICL complex by said test blockade agent.
35. A method of identifying an ICR/ICL blockade agent, comprising:
   (a) contacting:
      (i) an effector cell displaying on its surface PD-1, and comprising: T cell Receptor (TCR) and a nuclear factor of activated T cells (NFAT) promoter-dependent luciferase reporter, and
      (ii) an artificial antigen presenting cell (aAPC) displaying anti-cluster-of-differentiation-3 (CD3) antibody and PD-L1; and
   (b) detecting a signal from said NFAT-promoter-dependent luciferase reporter.
36. The method of paragraph 35, further comprising:
   (c) adding said test blockade agent; and
   (d) repeating detection of said signal from said reporter.
37. The method of paragraph 36, further comprising:
   (e) comparing said signal from step (b) with said signal from step (d), wherein a gain of signal from step (b) to step (d) indicates inhibition of said ICR/ICL complex by said test blockade agent.
38. A method comprising administering an artificial antigen presenting cell (aAPC) displaying on its surface a T cell receptor (TCR) activator and an immune checkpoint ligand (ICL) to a system comprising a natural effector cell.
39. A method comprising administering an artificial antigen presenting cell (aAPC) displaying on its surface a T cell receptor (TCR) activator and an immune checkpoint ligand (ICL) to a system comprising an artificial effector cell.

## Claims

1. A composition comprising:
(a) an effector cell displaying on its surface i) one or two immune checkpoint receptors (ICR) and ii) a T-cell receptor (TCR) and iii) a CD28 and comprising a gene encoding a fluorescent or bioluminescent reporter protein, the expression of which is under control of an IL-2 promoter or a nuclear factor-kappa B (NF-κB) promoter; and,
(b) an antigen presenting cell (APC) displaying on its surface: i) a T cell receptor (TCR) activator and ii) one or two immune checkpoint ligands (ICL), wherein the TCR activator is an anti-cluster-of-differentiation-3 (CD3) antibody or a fragment thereof comprising a Fab domain;
wherein the ICR and ICL form an ICR/ICL complex upon interaction, and wherein formation of the ICR/ICL complex results in altered expression of the fluorescent or bioluminescent reporter protein.

2. The composition of claim 1, wherein the ICL is selected from the group consisting of CD80/86, PD-L1, PD-L2, B7-H4, CD155, CD112, HLA-G, galectin-9, and HVEM.

3. The composition of claim 1 or claim 2, wherein the ICR is selected from the group consisting of PD-1, CTLA-4, LAG-3, TIM-3, CD160, TIGIT, CD112R, ILT2, ILT4, IL-10 receptor, and BTLA.

4. A system comprising:
(a) an effector cell displaying on its surface i) one or two immune checkpoint receptors (ICR), and ii) a T cell receptor (TCR) and iii) a CD28 and comprising a gene encoding a fluorescent or bioluminescent reporter protein, the expression of which is under control of an IL-2 promoter or a nuclear factor-kappa B (NF-κB) promoter; and
(b) an antigen presenting cell (APC) displaying on its surface: (i) a T cell receptor (TCR) activator and (ii) one or two immune checkpoint ligands (ICL), wherein the TCR activator is an anti-cluster-of-differentiation-3 (CD3) antibody or a fragment thereof comprising a Fab domain;
wherein the ICR and ICL form an ICR/ICL complex upon interaction, and wherein formation of the ICR/ICL complex results in altered expression of the fluorescent or bioluminescent reporter protein.

5. The system of claim 4, further comprising
(i) a blockade agent or test blockade agent, optionally wherein (a) the blockade agent inhibits formation of the ICR/ICL complex resulting in modulation of TCR activation, CD28 activation or TCR pathway activation, or (b) the blockade agent or test blockade agent is a small molecule, peptide, protein, or antibody, or
wherein
(ii) the ICL is selected from the group consisting of CD80/86, PD-L1, PD-L2, B7-H4, CD155, CD112, HLA-G, galectin-9, and HVEM, or
(iii) the ICR is selected from the group consisting of PD-1, CTLA-4, LAG-3, TIM-3, CD160, TIGIT, CD112R, ILT2, ILT4, IL-10 receptor, and BTLA, or
(iv) the effector cell is selected from T cells including Jurkat, HuT-78, CEM, Molt-4, and primary T cells.

6. The system of claim 5, wherein formation of the ICR/ICL complex results in modulation of TCR activation by the TCR activator and/or modulation of one or more TCR-dependent pathways.

7. The system of any one of claims 4 to 6, wherein the bioluminescent reporter protein is a luciferase.

8. A system comprising:
(a) an effector cell comprising TCR and PD-1 on its surface, and an IL-2 promoter-dependent luciferase; and
(b) an artificial antigen presenting cell (aAPC) displaying on its surface: anti-CD3 antibody or antibody fragment comprising a Fab domain, and PD-L1,
wherein formation of a PD-1/PD-L1 complex results in a suppressed expression of the IL-2-promoter-dependent luciferase.

9. The system of claim 8, wherein the system further comprises a blockade agent or test blockade agent.

10. A method for identifying a blockade agent, comprising:
(a) producing a system according to any one of claims 4 to 9, wherein production of the composition comprises co-culturing an effector cell defined in any one of claims 1 to 9 and an antigen-presenting cell defined in any one of claims 1 to 9; and
(b) detecting a fluorescent or bioluminescent signal from said fluorescent or bioluminescent reporter protein.

11. The method of claim 10, further comprising adding to the system a blockade agent, wherein the blockade agent inhibits formation of the ICR/ICL complex or inhibits ICR/ICL-dependent modulation of TCR activation by the TCR activator and/or modulation of one or more TCR-dependent pathways, and/or modulation of CD28 activation by CD80/CD86.

12. The method of claim 10, wherein said TCR-pathway-dependent reporter or a signal from said reporter is detected: (1) before, (2) concurrent with, and/or (3) after addition of said blockade agent, optionally wherein the method further comprises a step of comparing the signal from (1) before, (2) concurrent with, and/or (3) after addition of said blockade agent to determine the effect of the blockade agent.

13. A method comprising:
(a) co-culturing:
(i) an effector cell displaying a T cell Receptor (TCR) and one or two immune checkpoint receptors (ICR) on its surface, and comprising a gene encoding a fluorescent or bioluminescent reporter protein, the expression of which is under control of an IL-2 promoter or a nuclear factor-kappa B (NF-κB) promoter; and
(ii) an antigen presenting cell (APC) displaying a TCR activator and one or two immune checkpoint ligands (ICL), wherein the TCR activator is an anti-cluster-of-differentiation-3 (CD3) antibody or a fragment thereof comprising a Fab domain, wherein the ICR and ICL form an ICR/ICL complex upon interaction;
(b) adding a test blockade agent;
(c) detecting a signal from said reporter; and
(d) comparing said signal from said reporter with a control, wherein a gain of signal with respect to the control indicates inhibition of said ICR/ICL complex by said test blockade agent.

14. A method of identifying an ICR/ICL blockade agent, comprising:
(a) co-culturing:
(i) an effector cell displaying T cell Receptor (TCR) and one or two immune checkpoint receptors (ICR) on its surface, and comprising a gene encoding a fluorescent or bioluminescent reporter protein, the expression of which is under control of an IL-2 promoter or a nuclear factor-kappa B (NF-κB) promoter; and
(ii) an antigen presenting cell (APC) displaying anti-cluster-of-differentiation-3 (CD3) antibody and an immune checkpoint ligand (ICL), wherein the ICR and ICL form an ICR/ICL complex upon interaction;
(b) detecting a signal from said reporter;
(c) adding a test blockade agent;
(d) repeating detection of said signal from said reporter; and
(e) comparing said signal from step (b) with said signal from step (d), wherein a gain of signal from step (b) to step (d) indicates inhibition of said ICR/ICL complex by said test blockade agent.


SEQUENCE LISTING

<110>  PROMEGA CORPORATION

<120>  SYSTEMS AND METHODS FOR ASSESSING MODULATORS OF IMMUNE
       CHECKPOINTS

<130>  PROBH/P63466EP

<150>  US 62/082,458
<151>  2014-11-20

<150>  PCT/US2015/061906
<151>  2015-11-20

<160>  1

<170>  PatentIn version 3.5

<210>  1
<211>  30
<212>  DNA
<213>  Artificial sequence

<220>
<223>  NFAT response element

<400>  1
ggaggaaaaa ctgtttcata cagaaggcgt                                        30